# EUROPEAN PATENT APPLICATION

(11) **EP 3 792 347 A1**
(43) Date of publication of application: **17.03.2021**
(21) Application number: 19799779.4
(22) Date of filing: 08.05.2019
(51) Int. Cl.: C12N 5/10, C07D 417/04, C07D 417/14, C12N 15/09, C12N 15/85, C12Q 1/04

(54) **METHOD FOR PRODUCING HOMOZYGOUS CELLS**

(30) Priority: 08.05.2018 JP 2018089779
(71) Applicant: Osaka University, Suita-shi, Osaka 565-0871 (JP)
(72) Inventor: YOSHIMURA, Yasuhide, Suita-shi, Osaka 565-0871 (JP); TAKEDA, Junji, Suita-shi, Osaka 565-0871 (JP)
(74) Representative: Hoffmann Eitle
(86) International application number: PCT/JP2019/018379
(87) International publication number: WO 2019/216338

(57) **Abstract**

The purpose of the present invention is to provide a technique by which a site-specific homozygote can be efficiently acquired by increasing cross efficiency and a homozygote in a genome modification-free state can be easily obtained. A method for producing homozygous cells, said method including: (A) a step for, into cells having a heterozygous mutation at a target site, introducing heterozygouse allele specific cleavage of DNA double strand of homologous chromosomes and thus inducing crossing in the presence of a Bloom's syndrome protein inhibitor to thereby give homozygous cells at the target site; and (B) a step for selecting the homozygous cells.

SEQUENCE LISTING

<110>  OSAKA UNIVERSITY

<120>  Method for Preparing  Homozygous Cells

<130>  19016WO

<150>  JP2018-089779
<151>  2018-05-08

<160>  5

<170>  PatentIn version 3.5

<210>  1
<211>  20
<212>  DNA
<213>  Artificial Sequence

<220>
<223>  gRNA targeting sequence for Chr19_ 34512783-34512803

<400>  1
cctggtgtgg gggccctgcg                                                   20


<210>  2
<211>  20
<212>  DNA
<213>  Artificial Sequence

<220>
<223>  gRNA targeting sequence for Chr19_40498170-40498189

<400>  2
gtcagggatg ggattaggga                                                   20


<210>  3
<211>  20
<212>  DNA
<213>  Artificial Sequence

<220>
<223>  gRNA targeting sequence for Chr19_44500571-44500590

<400>  3
cccagaagcc tccgcggcgc                                                   20


<210>  4
<211>  20
<212>  DNA
<213>  Artificial Sequence

<220>
<223>  gRNA targeting sequence for Chr19_44536861-44536880

<400>  4
attgttatat tggtgagggg                                                   20

<210>  5
<211>  20
<212>  DNA
<213>  Artificial Sequence

<220>
<223>  gRNA targeting sequence for Chr6_2810497-2810516

<400>  5
attgctttga tgctgggtca                                                    20

## Description

### TECHNICAL FIELD

The present invention relates to a method for producing homozygous cells. More specifically, the present invention relates to a method for efficiently producing homozygous cells.

### BACKGROUND ART

Our genome is composed of a pair of chromosomes, one of which is from the mother, and the other of which is from the father. Genomic sequence information is not identical between the maternal one and the paternal one (this state is called heterozygote), and single nucleotide polymorphism (SNP) exclusively is reported to exist at about one million positions on the genome. Besides SNP, more polymorphisms such as deletion and repeat sequences further exist. Heretofore, there have been a number of reports indicating that part of polymorphisms is related to disease. More recently, a lot of data suggesting the correlation between polymorphism and disease or trait has been reported in association with the dramatical advance in the whole genome sequencing technique and the analytical method thereof.

These data indicate the correlation between the polymorphism and the actual phenotype (disease or trait) statistically, but do not technically demonstrate the correlation. In order to technically demonstrate whether the polymorphisms are directly involved in human disease, it is necessary to analyze the phenotype for the homozygote of the polymorphism in question. The technique for changing a chromosome from a heterozygote to a homozygote has been conventionally performed as a mating experiment of laboratory animals or the like. In the case of human, since a mating experiment is impossible for ethical reasons, the only available method is to cause homologous recombination between the paternal chromosome and the maternal chromosome in a somatic cell such as an induced Pluripotent Stem cell (iPS cell), and acquire a cell strain in which part of chromosomes is changed to a homozygote.

A CRISPR/Cas9 method (Patent Document 1) is a technique in which a Cas9 nuclease which is a DNA cleaving enzyme, and a guide RNA vector expressed with a human U6 polymerase III promoter are co-expressed, to cleave a specific target DNA sequence. In other words, the CRISPR/Cas9 method is capable of introducing DNA double strand break (DSB) site-specifically. Once DSB has been introduced by the Cas9 nuclease, repair can be made through the homologous recombination repair pathway. Therefore, the CRISPR/Cas9 method has a potential as a tool capable of increasing the homologous recombination speed site-specifically.

### PRIOR ART DOCUMENT

### PATENT DOCUMENT

Patent Document 1: US Patent No. 8,697,359

### SUMMARY OF THE INVENTION

### PROBLEMS TO BE SOLVED BY THE INVENTION

The system of homologous recombination (crossover) is incorporated in meiosis, and the frequency of crossover is once or twice per each chromosome, which is relatively high. On the other hand, the frequency of crossover in a somatic cell is very low, and it has been difficult to systematically acquire a cell strain of a homologous recombinant having undergone site-specific crossover. The present inventors found that by combining suppression of gene expression of Bloom's syndrome protein (BLM protein) related to chromosome stability, and site-specific introduction of DNA double strand break (DSB) by CRISPR/Cas9 using a tetracycline gene expression modulating system (Tet-off), it is possible to efficiently acquire a site-specific homozygote in the aimed region of the chromosome.

However, since the cells of the homozygote obtained in this manner are produced by exogenously introducing the *BLM gene* expression regulation, the genome has undergone modification (namely, change in genetic information). As a technique of removing the genetically introduced part after recombination, use of a transposon vector is known. However, genetically incorporating the Tet-off into which the *BLM* gene expression regulation is introduced into a transposon vector would be technically and practically impossible for adverse effects on the gene expression of the transposon recognition sequence. In other words, the present inventor confronted a new problem that even if a site-specific homozygote is efficiently obtained by suppressing gene expression of BLM protein, the site-specific homozygote cannot be applied to therapy due to the impossibility of removing genome modification.

In light of the above, it is a primary object of the present invention is to provide a technique by which a site-specific homozygote having efficiently undergone homologous recombination can be acquired by increasing the crossover efficiency and a homozygote can be easily obtained in the form free of genome modification.

### MEANS FOR SOLVING THE PROBLEM

As a result of diligent efforts, the present inventor found that by introducing DNA double strand break that is specific to one allele of homologous chromosomes in the presence of a BLM protein inhibitor, a site-specific homozygote having efficiently undergone homologous recombination due to enhanced crossover efficiency can be acquired, and the acquired homozygote is obtained in such a state that genome modification is easy to remove therefrom. The present invention was accomplished by further repeating examinations on the basis of such findings.

That is, the present invention provides the invention of the following aspects.
Item 1. A method for producing homozygous cells, comprising:
   a step (A) of introducing DNA double strand break that is specific to one allele of homologous chromosomes into cells having a heterozygous mutation at a target site, and causing crossover in the presence of a Bloom's syndrome protein inhibitor to thereby obtain homozygous cells at the target site; and
   a step (B) of selecting the homozygous cells.
Item 2. The method according to item 1, wherein the Bloom's syndrome protein inhibitor is 1-(4-fluoro-3-(trifluoromethyl)phenyl)-3-(5-(pyridin-4-yl)1,3,4-thiadiazol-2-yl) urea and/or 1-(4-(1H-pyrazolyl)-3-cyanophenyl)-3-(5-(pyridin-4-yl)1,3,4-thiadiazol-2-yl) urea.
Item 3. The method according to item 1 or 2, wherein the Bloom's syndrome protein inhibitor is used in a concentration of 3.0 to 50.0 µM.
Item 4. The method according to any one of items 1 to 3, wherein the Bloom's syndrome protein inhibitor is made to coexist with the cells having a heterozygous mutation before the step (A).
Item 5. The method according to any one of items 1 to 4, wherein in the step (A), the DNA double strand break is conducted by a DNA double strand breaking method selected from a CRISPR/CRISPR-related nuclease method, a zinc finger nuclease (ZFN) method, or a transcription activator-like effector nuclease (TALEN) method.
Item 6. The method according to any one of items 1 to 5, wherein in the step (A), the introduction of DNA double strand break that is specific to one allele of homologous chromosomes is conducted by introducing a vector designed by selecting a targeting sequence of which polymorphism exists in one allele from targeting sequences in the DNA double strand break, into the cells having a heterozygous mutation.
Item 7. The method according to any one of items 1 to 6, wherein
   in the step (A), one or both of alleles of the homologous chromosomes is subjected to introduction of a selection cassette having such a sequence that a drug resistance gene to a first drug and a drug resistance gene to a second drug are arranged in directions opposite to each other, and inversion of the sequence, and
   in the step (B), cells that are resistant to both of the first drug and the second drug are selected as homozygous cells at the target site.
Item 8. The method according to item 7, wherein
   in the step (A), the selection cassette is introduced while it is incorporated in a transposon vector, and
   after the step (B), the selection cassette is removed by a re-excising transposase.
Item 9. The method according to item 7 or 8, wherein in the step (A), an insulator sequence is introduced on both sides or on either side of the selection cassette.
Item 10. The method according to any one of items 1 to 9, wherein the target site is a gene locus of a major histocompatibility antigen.
Item 11. The method according to any one of items 1 to 10, wherein the cells having a heterozygous mutation at the target site are diploid cells.
Item 12. The method according to item 11, wherein the diploid cells are human-derived cells.

### ADVANTAGES OF THE INVENTION

According to the present invention, since it is possible to acquire a site-specific homozygote having efficiently undergone homologous recombination due to enhanced crossover efficiency, and obtain the acquired homozygote in such a state that genome modification is easy to remove therefrom, the acquired homozygote is highly valuable, for example, in application to therapy by implantation and the like.

### BRIEF DESCRIPTION OF THE DRAWINGS

Fig. 1 schematically shows one example of a step (A) of the method for producing homozygous cells of the present invention.
Fig. 2 schematically shows another example of the step (A) of the method for producing homozygous cells of the present invention.
Fig. 3 schematically illustrates homologous recombination (crossover) by DNA double strand break (DSB) and a Bloom's protein (BLM protein) inhibitor.
Fig. 4 schematically shows a construction method of a double drug selection cassette.
Fig. 5 schematically shows a selection step when a double drug selection cassette is used.
Fig. 6 shows an experimental design of Reference example, and shows targeting of the Tet cassette for both alleles of the *BLM* gene locus by TALEN.
Fig. 7 shows an experimental design of Reference example, and shows targeting of the double selection cassette for the AAVS1 gene locus.
Fig. 8 shows an experimental design of Reference example and Examples, and shows a schematic view of the mitotic crossover in the 4N phase.
Fig. 9 shows the result of competitive PCR conducted for screening homozygous cells in chromosome 19 in Reference example using Tet off.
Fig. 10 shows the result of competitive PCR conducted for screening homozygous cells in chromosome 19 in Example using ML216.
Fig. 11 shows the result of competitive PCR conducted for screening homozygous cells in chromosome 6 in Example using ML216.
Fig. 12 shows the crossover efficiency of Reference example using Tet off (sample of 3 in the drawing) together with the crossover efficiencies (examined seven times for each sample) of the cases (samples 1, 2 in the drawing) where various conditions (presence or absence of Dox, and presence or absence of CRISPR) are varied in the reference example.
Fig. 13 shows the crossover efficiency of Example using ML216 (sample of 3 in the drawing) together with the crossover efficiencies (examined four times for each sample) of the cases (samples 1, 2 in the drawing) where various conditions (presence or absence of ML216, and presence or absence of CRISPR) are varied in the Example.
Fig. 14 shows distribution of crossover sites in chromosome 19 (Reference example).
Fig. 15 shows distribution of crossover sites at 9 Mb (35M region) from centromere of chromosome 19 (Reference example).
Fig. 16 shows distribution of crossover sites at 14 Mb (40M region) from centromere of chromosome 19 (Reference example).
Fig. 17 shows distribution of crossover sites at 19Mb-I, 19Mb-II(45M-1, 45M-2) from centromere of chromosome 19 (Reference example).
Fig. 18 shows distribution of crossover sites at 9 Mb (35M region) from centromere of chromosome 19 under suppression of BLM protein by ML216.
Fig. 19 schematically shows the short arm of chromosome 6 before crossover.
Fig. 20 shows a schematic view of the short arm of chromosome 6 after crossover.
Fig. 21 shows a flow cytometry profile of HLA-A haplotype in hiPSC (Example).
Fig. 22A shows the result of SNP array analysis in chromosome 6 and chromosome 19 for parental fibroblast cells.
Fig. 22B shows the result of SNP array analysis in chromosome 6 and chromosome 19 for human induced pluripotent stem cells (hiPSCs).
Fig. 22C shows the result of SNP array analysis in chromosome 6 and chromosome 19 for hiPSC-BLM ^{tet/tet}AAVS1 ^{cNP/+} obtained in the item (4) of Reference example.
Fig. 22D shows the result of SNP array analysis in chromosome 6 and chromosome 19 for homozygous cells hiPSC-BLM ^{tet/tet}AAVS1 ^{cNP/+}Dox(+)9M-CRISPR(+) obtained in Reference example.
Fig. 22E shows the result of SNP array analysis in chromosome 6 and chromosome 19 for homozygous cells hiPSC-AAVSl ^{cNP/+}ML216(+)9M-CRISPR(+) obtained in Example.
Fig. 22F shows the result of SNP array analysis in chromosome 6 and chromosome 19 for homozygous cells hiPSC-telHLA ^{cNP/+}ML216(+)HLA I-III-CRISPR(+) obtained in Example.

### EMBODIMENTS OF THE INVENTION

### [Method for producing homozygous cells]

A method for producing homozygous cells of the present invention includes a step (A) of introducing DNA double strand break that is specific to one allele of homologous chromosomes into cells having a heterozygous mutation at a target site, and causing crossover in the presence of a Bloom's syndrome protein inhibitor to thereby obtain homozygous cells at the target site, and a step (B) of selecting the homozygous cells.

### [Step (A)]

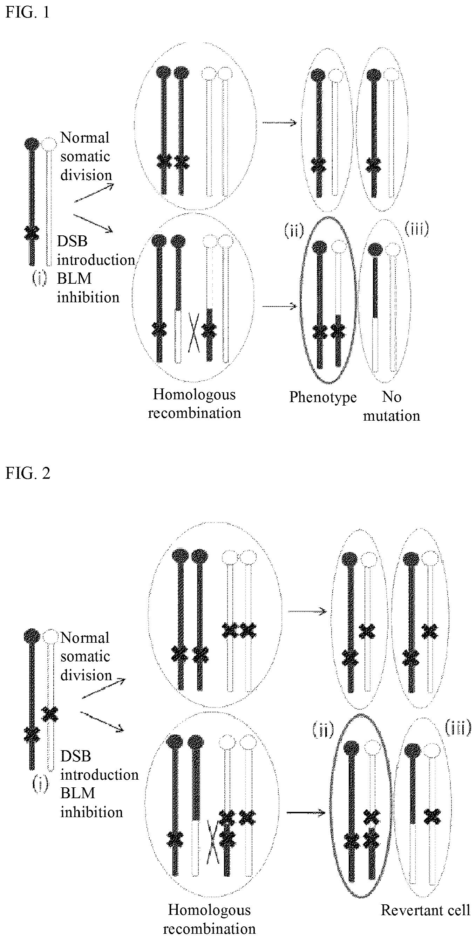

Fig. 1 and Fig. 2 schematically show examples of the step (A). In the step (A), by introducing DNA double strand break that is specific to one allele of homologous chromosomes (DSB) ex vivo into cells (indicated by (i) in Fig. 1 and Fig. 2) having a heterozygous mutation (shown by X in Fig. 1 and Fig. 2) at a target site and causing crossover in the presence of a Bloom's syndrome (BLM) protein inhibitor, homozygous cells at the target site (indicated by (ii) and (iii) in Fig. 1 and Fig. 2) are obtained by the loss of heterozygosity (LOH).

### [Target site]

The target site in the present invention is a site containing a heterozygous mutation, and is a site where homozygosis is to be caused by crossover on chromosomes. Examples of the heterozygous mutation include a mutation that can cause a disease, and a mutation that can generate individual difference in immunological reactivity. Examples of the mutation include, but are not particularly limited to, single-nucleotide polymorphism (SNP), insertion/deletion (Indel), and copy number variation (CNV). The target site may contain only one mutation, or a plurality of mutation. The length of the target site is not particularly limited, and may be, for example, 1 to 200 Mbp including a narrow region on a chromosome, and a broad region such as the entire short arm or the entire long arm.

One specific example of the target site is a site suspected of existence of a mutation that causes a disease by a linkage analysis or the like. Preferably, an intergenic region is recited. By causing crossover aiming at such a target site by the present invention, two types of homozygous cells are obtained. One type is a homozygote of the mutation (the cell indicated by (ii) in Fig. 1), and the cell can be applied for technically analyzing the relationship between the mutation and disease. This cell is useful, particularly in searching for causal factors in intergenic regions in multifactorial disorders. The other type is a homozygote having lost the mutation and thus having a normal phenotype (the cell indicated by (iii) in Fig. 1), and this cell can be applied to regenerative medicine. In the case where the mutation that causes disease is known, artificial reversion mosaics as indicated by (ii) and (iii) in Fig. 2 are generated as homologous recombinants by crossover aiming at the target site, and thus a revertant cell as indicated by (iii) in Fig. 2 can be obtained. Although the cell indicated by (iii) in Fig. 1 also corresponds to a revertant cell, the revertant cell indicated by (iii) in Fig. 2, in particular, can be applied to therapy, analysis of disease development mechanism, analysis of influence on a living body at the time of recovery from genetic mutation in different cell series and so on.

Further, other specific examples of the target site include gene loci of a major histocompatibility antigen defining MHC (Major Histocompatibility Complex), preferably HLA (Human Leukocyte Antigen). By causing crossover aiming at such a target site by the present invention, and transforming into a homozygote of the mutation (the cell indicated by (ii) in Fig. 1), it is possible to produce cells that are suited as regenerative medial materials, vaccines for preventing onset of cancer, and cancer immune drugs for suppressing recurrence after cancer resection on a patient with cancer onset. Also, since various sites can be selected as a target site where crossover is to be caused by the present invention, it is possible to produce MHC homozygous cells of various haplotypes. The variety of MHC homozygous cells provided by the present invention makes it possible to construct a cell bank composed of MHC homozygous cells. The homozygous cells stocked in such a cell bank, when used as a regenerative medical material, are desirable in that immunological adaptability of the regenerative medial material is improved owing to the variety of the homozygous cells. Alternatively, the homozygous cells stocked in such a cell bank, when used as a cancer vaccine or a cancer immune drug, are desirable in that cells that are immunologically adaptable to the patient can be rapidly selected from the cell bank and applied to the therapy owing to the variety of the homozygous cells without the need for producing own iPS cells (cancer vaccine or cancer immune drug) from the patient himself/herself. The gene regions defining HLA are located in the short arm of chromosome 6, and examples of the gene regions include class I (HLA-A, -B, -C, etc.), class II (HLA-DR, - DQ, -DP, etc.), and class III. Among these gene regions, a target site may be set in one region, a target site may be set so that it spans sequential two regions, or a target site may be set so that it spans all the regions (namely, all the regions from class I to class II).

### [Cells]

The kind of cells including a heterozygous mutation in a target site is not particularly limited. Since the present invention is capable of enhancing the crossover efficiency, it is especially useful when the heterozygous cells are diploid cells in which crossover is originally (under the condition that BLM is not suppressed) unlikely to occur. Examples of the diploid cells include pluripotent stem cells, somatic stem cells, blast cells, and somatic cells. Examples of the pluripotent stem cells include ES cells, EG cells, mGS cells, and iPS cells; examples of the somatic stem cells include mesenchymal stem cells, hematopoietic stem cells (bone marrow cells, peripheral stem cells, cord blood stem cells, etc.), vascular stem cells (vascular progenitor cells, vascular endothelial progenitor cells, etc.), and neural stem cells; examples of the blast cells include fibroblast cells, myoblast cells, and osteoblast cells; and examples of the somatic cells include corneal epithelial cells, oral mucosal epithelial cells, hepatic cells, thyroid cells, and blood cells (mononuclear cells, dendritic cells, etc.). The somatic stem cells are particularly useful in generating artificial reversion mosaics, and the pluripotent stem cells are particularly useful in making HLA homologous to enhance the versatility in implantation.

The origin of the heterozygous cells is not particularly limited, and cells derived, for example, from mammals (e.g., murine, rat, swine, bovine, and primates such as human beings and common marmosets), Xenopus laevis, zebrafish, fruit fly, nematode, plants and so on can be recited. When the heterozygous cells are somatic cells, the origin of the cells is preferably mammals, more preferably human beings.

### [Bloom's syndrome protein inhibitor]

Bloom's syndrome is known as a disease accompanied by recessive mutation in the Bloom's gene, and symptoms of facial telangiectasia, photosensitivity, dwarfism, immune deficiency, high-frequency carcinogenesis and the like are reported. Bloom's syndrome protein (BLM protein) is a protein associated with Bloom's syndrome, and as a Bloom's syndrome protein inhibitor in the present invention, a substance that inhibits helicase activity of BLM protein is used. Specific examples include a 5-(pyridin-4-yl)-1,3,4-thiadiazole-2-amine derivative. Examples of the 5-(pyridin-4-yl)-13,4-thiadiazol-2-amine derivative include 1-phenyl-3-(5-(pyridin-4-yl)1,3,4-thiadiazol-2-yl)urea derivative, and more specific examples include 1-(4-fluoro-3-(trifluoromethyl)phenyl)-3-(5-(pyridin-4-yl)1,3,4-thiadiazol-2-yl) urea (ML216; compound (1) of the following formula) and 1-(4-(1H-pyrazolyl)-3-cyanophenyl)-3-(5-(pyridin-4-yl)1,3,4-thiadiazol-2-yl) urea (compound (2) of the following formula). One kind of these BLM protein inhibitors may be used alone, or a combination of a plurality of kinds thereof may be used.

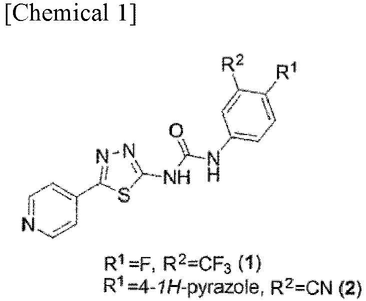

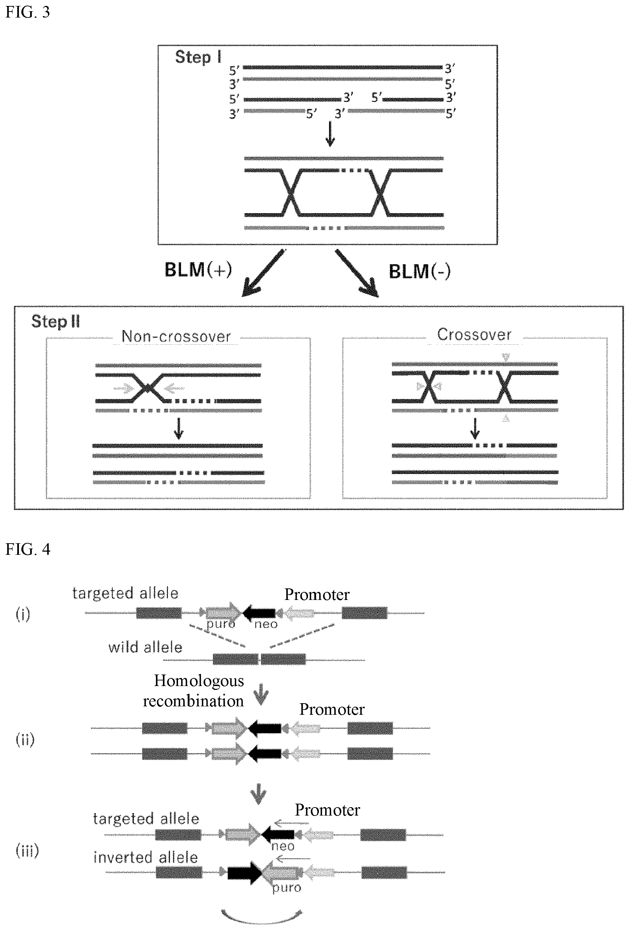

In the present invention, the BLM protein inhibitor enables homologous recombination to occur efficiently by crossover after DNA double strand break (DSB). Fig. 3 schematically illustrates crossover by DSB and the BLM protein inhibitor. As shown in Step I of Fig. 3, as DSB occurs in DNA, the cleaved DNA is cut out in the direction of 5' → 3', and a single-stranded segment is generated. The single-stranded DNA segment finds the segment complementary to itself and forms a double Holliday junction as shown in Step II of Fig. 3. In the absence of a BLM protein inhibitor (BLM(+)), crossover reaction rarely occurs because DNA loosens by the helicase activity of the BLM protein (Non-crossover). On the other hand, in the presence of a BLM protein inhibitor (BLM(-)), it is considered that crossover reaction occurs because DNA fails to successfully loosen and DNAs bind to each other (Crossover).

In the step (A), the BLM protein inhibitor can be used in a concentration of, for example, 3.0 µM or more from the viewpoint of efficiently causing crossover. From the viewpoint of causing crossover more efficiently, or when the heterozygous cells are human iPS cells, in particular, the BLM protein inhibitor can be used in a concentration of preferably 10.0 µM or more, more preferably 11.0 µM or more, further preferably 12.0 µM or more. Also, from the viewpoint of causing crossover efficiently, the BLM protein inhibitor can be used in a concentration of, for example, 50.0 µM or less, preferably 25.0 µM or less. From the viewpoint of causing crossover more efficiently, or when the heterozygous cells are human iPS cells, in particular, the BLM protein inhibitor can be used in a concentration of, more preferably 14.0 µM, further preferably 13.5 µM, still further preferably 13.0 µM. Examples of the specific concentration range of the BLM protein inhibitor include 3.0-50.0 µM, 3.0-25.0 µM, 3.0-14.0 µM, 3.0-13.5 µM, 3.0-13.0 µM, 10.0-50.0 µM, 10.0-25.0 µM, 10.0-14.0 µM, 10.0-13.5 µM, 10.0-13.0 µM, 11.0-50.0 µM, 11.0-25.0 µM, 11.0-14.0 µM, 11.0-13.5 µM, 11.0-13.0 µM, 12.0-50.0 µM, 12.0-25.0 µM, 12.0-14.0 µM, 12.0-13.5 µM, and 12.0-13.0 µM. More specifically, the BLM protein inhibitor can be contained so that the final concentration of the BLM protein inhibitor is the aforementioned concentration per 10 mL of a culture solution charged with 10⁶ heterozygous cells.

From the viewpoint of causing transformation more efficiently, it is preferred to let the BLM protein inhibitor coexist with the heterozygous cells in the culture solution before the step (A), namely, before introduction of a nucleic acid construct for introducing DNA double strand break that is specific to one allele of homologous chromosomes into the heterozygous cells. Specifically, it is possible to culture the heterozygous cells in the presence of the BLM protein inhibitor. From the viewpoint of causing crossover more efficiently, the time during which the BLM protein inhibitor coexists before introduction of the nucleic acid construct is 6 to 24 hours, preferably 10 to 24 hours, more preferably 10 to 15 hours.

### [Nucleic acid construct for introducing DNA double strand break heterozygouse allele specifically]

The nucleic acid construct to be introduced into heterozygous cells is constructed by being incorporated into a targeting vector that actuates introduction of DNA double strand break that is specific to one allele of homologous chromosomes.

In the present invention, examples of the method for DNA double strand break include, but are not limited to, CRISPR-CRISPR-related nuclease method, a zinc finger nuclease (ZFN) method, or a transcriptional activator-like effector nuclease (TALEN) method, preferably CRISPR/CRISPR-related nuclease method. Examples of the CRISPR-related nuclease include, but are not limited to Cas3, Cas9, and Cpf1. A person skilled in the art can easily design a cassette for expressing such a DNA double strand break system according to the site where the DNA double strand break is to be made.

In order to construct the introduction of DNA double strand break to be specific to one allele of homologous chromosomes (only one allele of homologous chromosomes undergoes DNA double strand break), a cassette for expressing a DNA double strand break system is designed by selecting a recognition sequence of which polymorphism such as single nucleotide polymorphism exists in one allele among recognition sequences targeted by the DNA double strand break system for breakage. For example, a ZF domain recognition sequence in the case of using the ZFN, a TALE domain sequence in the case of using the TALEN method, and a protospacer adjacent motif (PAM) in the case of using the CRISPR method can be recited. The PAM sequence can be determined depending on the origin of the CRISPR-related nuclease to be used.

More specifically, when the ZFN method and the TALEN method are used, it is possible to select a recognition sequence of which polymorphism exists in one domain sequence of a pair of adjacent domain recognition sequences. When the CRISPR method is used, a PAM sequence in which single nucleotide polymorphism exists can be selected. In particular, when the PAM sequence is NGG, it is preferred to select the one in which single nucleotide polymorphism exists at the third nucleotide G as a recognition sequence in terms of more excellent one allele break specificity. As a specific method for selecting a recognition sequence of which polymorphism exists in one allele, a polymorphism detecting method based on a known genotyping may be used.

The target vector may have an appropriately designed selection cassette introduced therein to make the cells having undergone crossover selectable. The gene contained in the selection cassette can be any gene as long as it functions as an index for selecting host cells, and examples of the gene include a drug resistance gene, a fluorescent protein gene, a reporter gene, and a flanking probe gene.

Examples of the drug resistance gene include a puromycin resistance gene, a neomycin resistance gene, a hygromycin resistance gene, a zeocin resistance gene, an ampicillin resistance gene, a tetracycline resistance gene, a chloramphenicol resistance gene, and a blasticidin resistance gene.

In the present invention, it is preferred to use a selection cassette having such a sequence that a drug resistance gene to a first drug, and a drug resistance gene to a second drug are arranged in directions opposite to each other (double drug selection cassette). Fig. 4 schematically shows one example of a construction method of a double drug selection cassette. As shown in Fig. 4(i), by introducing a double drug selection cassette having such a sequence that a neomycin resistance gene (neo: a drug resistance gene to a first drug arranged in the expression direction) and a puromycin resistance gene (puro: a drug resistance gene to a second drug arranged in the direction opposite to the expression direction) are arranged in directions opposite to each other (neo/puro cassette) into one or the other of alleles of homologous chromosomes (targeted allele), the double drug selection cassette is introduced into both of the alleles when the homozygote is generated by crossover as shown in Fig. 4(ii). Further, as shown in Fig. 4(iii), by inverting the sequence (the sequence in which the drug resistance gene to the first drug and the drug resistance gene to the second drug are arranged in directions opposite to each other), only the homozygote generated by crossover becomes resistant to both of the first drug and the second drug. To be more specific, by introducing a targeting vector constructed so that the sequence is arranged between two loxP sequences arranged in directions opposite to each other, into heterozygous cells (Fig. 4(i)) to induce crossover by DNA double strand break (Fig. 4(ii)), it is possible to invert the sequence between the loxP sequences under the action of a Cre recombinase (Fig. 4(iii)).

Examples of the fluorescent protein gene include genes encoding GFP, YFP, CFP, BFP, Venus, DsRed, HcRed, AsRed, ZsGreen, ZsYellow, AmCyan, AcGFP, Kaede and the like fluorescent proteins. Examples of the reporter gene include a luciferase gene, and β-galactosidase. Regarding the flanking probe gene, a person skilled in the art can appropriately determine any sequence that can be used as a detection probe when homologous recombination is examined by the Southern blotting method.

In the present invention, it is preferred that the selection cassette is introduced while it is incorporated in a transposon vector. More specifically, a transposon vector incorporating a selection cassette in such a manner that the selection cassette and a promoter are sandwiched between two transposon recognition sequences (see Fig. 4), and a re-excising transposase expression vector are co-introduced into heterozygous cells, and the selection cassette can be incorporated into genome by a transposase expressed in the cells. The selection cassette thus incorporated can be removed from the genome with a re-excising transposase after completion of the later-described step (B). Use of the transposon expression system as described above is desired because the homozygotes can be obtained in the form free of genome modification.

When the target site is set in the HLA region, it is preferred that the selection cassette is inserted in a region closer to telomere than the target region. When the selection cassette is inserted into a site where inactivation by methylation or the like can occur, including the aforementioned region, it is preferred to introduce an insulator sequence on both sides or on either side of the selection cassette, preferably on both sides of the selection cassette from the viewpoint of suppressing inactivation. An insulator sequence is known as a sequence that keeps the independency of gene expression by functioning as a regulatory element that blocks transcription of a gene located within the activity range of the insulator sequence, but does not fluctuate the gene expression. Non-limiting examples of the insulator sequence include the sequence of positions 39519983 to 39520225 of human chromosome 7, and insulator sequences described in Genomic discovery of potent chromatin insulators for human gene therapy.Liu M, Maurano MT, Wang H, Qi H, Song CZ, Navas PA, Emery DW, Stamatoyannopoulos JA, Stamatoyannopoulos G.Nat Biotechnol. 2015 Feb; 33(2): 198-203., most preferably the sequence of positions 39519983 to 39520225 of human chromosome 7.

The above-described exogenous DNA can be incorporated into the targeting vector as an expression unit in which the DNA is appropriately combined with sequences related to gene expression. Examples of the sequences related to expression of exogenous DNA include sequences essential for the gene expression such as a promoter sequence and a transcription termination signal sequence, and sequences that modulate the gene expression such as a modulation element. Examples of the promotor sequence include CMV, SV40, RSV, EF1α, CAG, U6, pGK and the like. Examples of the transcription termination signal sequence include a BGH polyA signal sequence, and a SV40 polyA signal sequence. Examples of the modulation elements include an enhancer, an IRES (internal ribosome entry site) sequence, a loxP sequence and a FRT sequence.

The type of the targeting vector is not particularly limited, and for example, plasmid vectors, cosmid vector, fosmid vectors, viral vectors (adeno associated virus (AAV) vector, adenovirus vector, retrovirus vector, lentivirus vector), and artificial chromosome vectors (bacterial artificial chromosome (BAC), PI bacteriophage artificial chromosome (PAC), yeast artificial chromosome (YAC), human artificial chromosome (HAC) and so on) and the like can be recited.

The method for introducing a targeting vector into heterozygous cells is not particularly limited, and a known method can be used. For example, as a viral vector-based technique, a method of infecting with a viral vector of retrovirus, lentivirus, adeno associated virus or the like virus is recited, and as a non viral vector-based technique, a lipofection method, an eletroporation method, a microinjection method or the like physicochemical method can be recited.

After introduction of the targeting vector into heterozygous cells, incubation is conducted in the presence of a BLM protein inhibitor. During the incubation, DNA double strand break that is specific to one allele of homologous chromosomes (Step I in Fig. 3 described above) by expression of the nucleic acid construct in the presence of the BLM protein inhibitor, and subsequent crossover (Fig. 3 Step II described above) occur, and thus homozygous cells of the target part can be obtained as a homologous recombinant by LOH (Fig. 1 and Fig. 2 described above). The incubation time in the presence of the BLM protein inhibitor can be 20 hours or more, for example, 20 to 60 hours from the viewpoint of causing the homologous recombination by crossover more efficiently. The incubation time is preferably 40 to 60 hours, more preferably 45 to 60 hours from the viewpoint of causing the homologous recombination by crossover further efficiently.

### [Step (B)]

In the step (B), homozygous cells are selected. Any of the two types of homozygous cells generated as homologous recombinants by crossover may be selected. For selection, the cells may be cultured in a culture medium (selection culture medium) containing a selection marker suited for the content of the selection cassette used in the step (A). When a selection cassette containing a drug resistance gene is used, cells (positive clone) established in the culture solution containing a corresponding drug can be selected; when a selection cassette containing a fluorescent protein gene is used, selection can be made by observation under a fluorescent microscope; when a selection cassette containing a reporter gene is used, selection can be made by adding a corresponding substrate; and when a selection cassette containing a flanking probe gene is used, selection can be made by Southern blotting.

When a selection cassette having such a sequence that a drug resistance gene to a first drug, and a drug resistance gene to a second drug are arranged in directions opposite to each other (double drug selection cassette) is used as the selection cassette in the step (A), cells that are resistant to both of the first drug and the second drug can be selected as the objective homozygous cells.

For example, in the example of Fig. 4, puromycin resistance is acquired only after inversion of the double drug selection cassette by Cre recombinase. Therefore, initially, selection can be made by using the second drug (puromycin in the example of Fig. 4) corresponding the drug resistance gene (puromycin resistance gene in the example of Fig. 4) arranged in the direction opposite to the expression direction in the double drug selection cassette. Since it is supposed that the double drug selection cassette repeats inversion for a certain period of time by the Cre recombinase, selection can be made by using the first drug (neomycin in the example of Fig. 4) corresponding to the drug resistance gene (neomycin resistance gene in the example of Fig. 4) arranged in the expression direction in the double drug selection cassette in the certain period of time. For example, in the certain period of time, selection can be made by initially using the second drug, and then using both of the second drug and the first drug. Selection using both of the second drug and the first drug may be made twice or three or more times, and in this case, the concentration of the first drug can be increased with the number of times of selection.

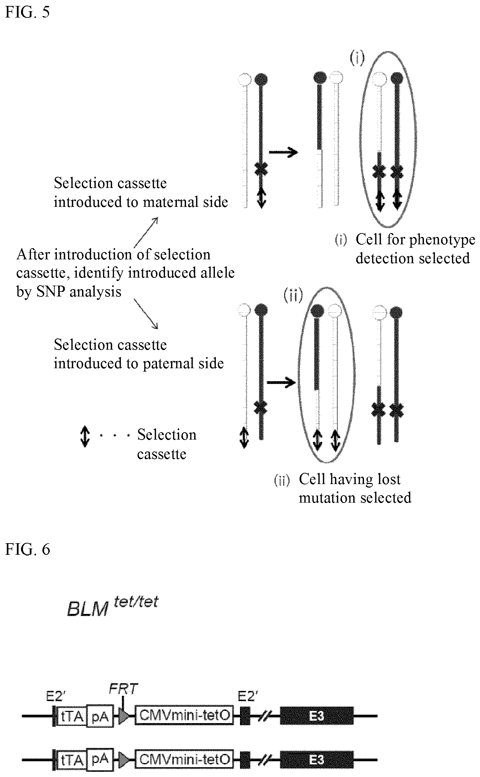

Fig. 5 more specifically illustrates a selection step when a double drug selection cassette is used. For example, in the case where a heterozygous mutation exists in one (indicated by maternal in the drawing) of alleles of homologous chromosomes in heterozygous cells, if the double drug selection cassette (indicated by the double-pointed arrow in the drawing) is introduced into the one (maternal) allele, the homozygous cells with homozygosity of the mutation as shown in (i) in the drawing, among the obtained two types of homozygous cells are selected as cells that are resistant to both of the first drug and the second drug. The cells can be applied to cells for detecting the phenotype. On the other hand, if the double drug selection cassette is introduced into the other (paternal) allele, the cells having lost the mutation as shown in (ii) in the drawing, among the obtained two types of homozygous cells are selected as cells that are resistant to both of the first drug and the second drug. The cells can be applied to cells for therapy.

### [Other steps]

The homozygous cells selected in the step (B) can be subjected to the step suited for their application purpose. For example, when the state of free of modification in genome is required, for example, in therapeutic application of the selected homozygous cells, the selection cassette incorporated into genome in the step (A) is removed. More specifically, by introducing a re-excising transposase expression vector to the selected homozygous cells, transposase is expressed in the cells, and the incorporated selection cassette can be removed without leaving any traces.

### [Homozygous cells]

The homozygous cells obtained by the present invention can be applied to a variety of uses depending on the target site that is made homologous. When the homozygous cells are obtained as a homozygote of a mutation that causes a disease (cells indicated by (ii) in Fig. 1), the cells can be applied to technically analyze the relationship between the mutation and the disease. This cell is useful, particularly in searching for causal factors in intergenic regions in multifactorial disorders. When the homozygous cells are obtained as cells having lost the mutation and having a normal phenotype (cells indicated by (iii) in Fig. 1), the cells can be applied to regenerative medicine. Further, when the homozygous cells are obtained as revertant cells (cells indicated by (iii) in Fig. 2), the cells can be applied to therapy, analysis of disease development mechanism, analysis of influence on a living body at the time of recovery from genetic mutation in different cell series and so on.

In particular, when homozygous cells are obtained as a MHC (major histocompatibility complex) such as HLA (human leukocyte antigen) that is made homologous, the homozygous cells can be suitably used as regenerative medical materials, vaccines for preventing onset of cancer, and cancer immune drugs for suppressing recurrence after cancer resection on a patient with cancer onset. Also, since various sites can be selected as a target site where crossover is to be caused by the present invention, it is possible to produce MHC homozygous cells of various haplotypes. The variety of MHC homozygous cells provided by the present invention makes it possible to construct a cell bank composed of MHC homozygous cells. The homozygous cells stocked in such a cell bank, when used as a regenerative medical material, are desirable in that immunological adaptability of the regenerative medial material is improved owing to the variety of the homozygous cells. Alternatively, the homozygous cells stocked in such a cell bank, when used as a cancer vaccine or a cancer immune drug, are desirable in that cells that are immunologically adaptable to the patient can be rapidly selected from the cell bank and applied to the therapy without the need for producing own iPS cells (cancer vaccine or cancer immune drug) from the patient himself/herself

### EXAMPLES

Hereinafter, the present invention is described more specifically by indicating Examples, however, the present invention is not limited to these.

### Outline

First, as Reference example, homozygous cells were produced by combining suppression of BLM protein gene expression using a tetracycline gene expression modulating system (Tet-off) without using a Bloom's syndrome protein inhibitor ML216, and introduction of a CRISPR/Cas9 that is designed to heterozygouse allele specifically introduce DNA double strand break (DSB). Further, as an Example, homozygous cells were produced by introducing a CRISPR/Cas9 that is designed to heterozygouse allele specifically introduce DSB, into cells in the presence of a Bloom's syndrome protein inhibitor ML216 without using Tet-off.

### [Reference example] Production of homozygous cells by construction of BLM gene regulatory unit and insertion into endogenous BLM gene using Tet-off system, and heterozygouse allele specific DNA double strand break

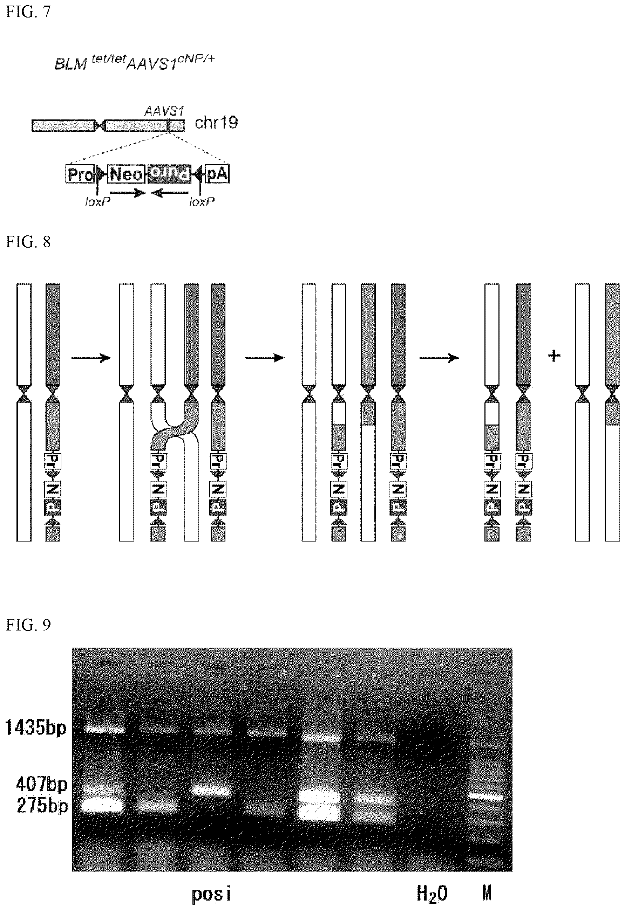

By conducting a double drug selection with neomycin and puromycin by combining a Tet-off system that temporarily shuts down the expression of the Bloom's gene, and introduction of a CRISPR/Cas9 that is designed to heterozygouse allele specifically introduce DNA double strand break (DSB) without using ML216, cell strains of site-specific homozygotes respectively aiming at the AAVS1 region of chromosome 19 (9 Mb from centromere (35M region), 14 Mb from centromere (40M region), and 19 Mb-I, 19 Mb-II from centromere (45M-1 region, 45M-2 region) as target sites were acquired. The 19 Mb-I, 19 Mb-II from centromere (45M-1 region, 45M-2 region) are different regions in 45M region. This experiment was designed so that expression of the Bloom's gene decreases only when a tetracycline derivative (doxycycline: Dox) was added. As a double drug selection cassette, the one shown in Fig. 4 was used. In the double drug selection cassette, two kinds of genes (neomycin and puromycin) enabling drug selection are arranged in directions opposite to each other, and oppositely-directed loxPs exist outside the two kinds of genes. The double drug selection cassette becomes two copies by changing into a site-specific homozygote, and becomes resistant to the two kinds of drugs by the action of a Cre recombinase. The experimental design is shown in Fig. 6 to Fig. 8. Fig. 6 shows targeting of the Tet cassette for both alleles of the *BLM* gene locus by TALEN. The puromycin selection cassette after the targeting was removed by a flippase recombinase. Fig. 7 shows targeting of the double selection cassette for the AAVS1 gene locus. Fig. 8 is a schematic view of the mitotic crossover in the 4N phase. Details of techniques of construction of CRISPR/Cas9 system for heterozygouse allele specific DSB introduction, cell culture, RNA extraction and cDNA synthesis, DNA extraction, screening of homozygous cells, determination of crossover site, and flow cytometry are the same with the techniques described in the later-described Example.

### (1. BLM-targeting vector)

For preparation of a human BLM-targeting vector, a human cytomegalovirus (CMV) promoter was changed to a hEF1α promoter, and the Kozak sequence for tTA translation was modified from AGGATT to GCCACC in a murine Blm targeting vector (Yusa K, Horie K, Kondoh G, Kouno M, Maeda Y, Kinoshita T, Takeda J. Genome-wide phenotype analysis in ES cells by regulated disruption of Bloom's syndrome gene. Nature. 2004 Jun 24; 429(6994): 896-9.). Further, an artificial intron sequence was removed from the murine Blm-targeting vector. The left and right arms of the targeting vector were prepared by PCR using the respective primers shown in the table below.

**[Table 1]**

| | |
|---|---|
| hBLMLarmF1 | TCTGTTTAAACAAGATCAATACTTTTAAACTGGATTCCAAA |
| hBLMLarmR1 | GGCTTAATTAAATAATCCTAAAAAGTGAGGGAAAAAGAAAT |
| hBLMRarmF1 | ATCTGCGGCCGCGATTATGGCTGCTGTTCCTCAAAATAATCT |
| hBLMRarmR3 | AGAAGGTGGAACAAAATCCGTATCATAATC |

### (2. Targeting vector for AAVS1 region of chromosome 19 having selection cassette)

A selection cassette (cNP cassette) was prepared by providing the cNP cassette used in mouse ESC(Horie, K., Kokubu, C., Yoshida, J., Akagi, K., Isotani, A., Oshitani, A., Yusa, K., Ikeda, R., Huang, Y., Bradley, A., et al. (2011). A homozygous mutant embryonic stem cell bank applicable for phenotype-driven genetic screening. Nature methods 8, 1071-1077.) with three modifications (changing Pgk promoter to hEF1α promoter, removing thymidine kinase gene, and replacing lox2272 with loxP).

Specifically, a selection plasmid was prepared in the following manner.

First, a bGH (bovine growth hormone) polyA sequence necessary for expression was amplified in the following conditions using the following template and primers.

### Template:

PGK-L21-NPT-L21-pA (A homozygous mutant embryonic stem cell bank applicable for phenotype-driven genetic screening. Nature Methods 8, 1071-1077 (2011))
Primers:
   *KpnI-bGHpA-puro-up-up:TAGTGGGCGCGCCGGTACCTTATCGAGTCC
   *Gly-puro-low:GGCTTGTACTCGGTCATGGT
      GGATCCAATAACTTCGTATAATGT
PCR conditions:
   94°C for 2 min, (98°C for 10 sec, 68°C for 1 min) × 35 cycles, and 68°C for 7 min

The puromycin gene was amplified in the following conditions using the following template and primers.
Template:
   pPGKpuro-F2F
Primers:
   *Gly -puro- up:GACCGAGTACAAGCC CACGGTGCGCCTCGC
   *puro-up:TAA CTCGAGTCATTA GGCACCGGGCTTGCGGGTCATGCAC
PCR conditions:
   94°C for 2 min, (98°C for 10 sec, 68°C for 1 min) x 35 cycles, and 68°C for 7 min

The neomycin gene was amplified in the following conditions using the following template and primers.
Template:
   pMulti-hEF1a-LP1-NPT-LP1-SLRarm
Primers:
   neo-low:TAATGACTCGAGTTAGAAGAACTCGTCAAGAAGGCGATAG
   XhoI-tTA-up:TCGATTAGTTCTCGAGCTTTTGGAGTACGT
PCR conditions:
   94°C for 2 min, (98°C for 10 sec, 68°C for 1.5 min) x 35 cycles, and 68°C for 7 min

The three fragments obtained in the manner as described above were introduced by In Fusion method into a basic vector having a hEF1α promoter and a targeting arm cloning site of Pmel/Ascl/Sfil and FseI/NotI inserted therein.

The left and right arms of the targeting vector were prepared by PCR using the primers shown in the table below.

**[Table 2]**

| | |
|---|---|
| AAVS1-LF1 | ATATGTTTAAACGAGCCAGGGGCATGAGATGGTGGACGAGGA |
| AAVS1-LR1 | ATATGGCGCGCCCACTAGGGACAGGATTGGTGACAGAAAAGC |
| AAVS1-RF1 | ATATGGCCGGGCGCCCCACTGTGGGGTGGAGGGGACAGATAA |
| AAVS1-RR1 | ATATATTTAAATTTCTCTGACCTGCATTCTCTCCCCTGGGCC |

### (3. Targeting of BLM region)

Using 8 µg of DNA (TALEN left 2 µg, TALEN right 2 µg, and BLM targeting vector 4 µg), one million human induced Pluripotent Stem cells (human iPS cells (hiPSCs); see the column of cell culture in later-described Example) in a 100 µl tip were transfected by electroporation (Neon transfection platform; Invitrogen) in the conditions of 1300 V, 30 msec, and 1 pulse. The TALEN sequence is as follows. The cells subjected to transfection were plated in a hESC culture medium containing 10 mM Y-27632 but not containing puromycin on a Synthemax^{(R)} II-SC-coated tissue culture plate. After two days from the transfection, selection was started with 0.5 µg/ml puromycin. Target clones were selected first by PCR using a primer hBLM-3 and a primer tTA2 shown in the table below, and then both allele target clones (hiPSC-BLM ^{tet/tet}) were selected by PCR using a primer hBLM-1, a primer hBLM-2 and a primer tTA-1.

**[Table 3]**

| | |
|---|---|
| hRLM-1 | TCCTATTACTCTCCCCACACTTCCAACAAT |
| hBLM-2 | TGGTAAGTTAGTGCCTATGTGACAGCAAAC |
| tTA-1 | GTATGCCGCCATTATTACGACAAGCTATCG |
| hBLM-3 | AAGTGGGACAAAAGCTGTATTATGATCATG |
| tTA-2 | GTGAGTATGGTGCCTATCTAACATCTCAAT |

**[Table 4]**

| |
|---|
| TALEN left: |
| gatctacgcacgctcggctacagccagcagcaacaggagaagatcaaaccgaaggttcgttcgacagtggcgcagcaccac gaggcactggtcggccatgggtttacacacgcgcacatcgttgcgctcagccaacacccggcagcgttagggaccgtcgctgtc aagtatcaggacatgatcgcagcgttgccagaggcgacacacgaagcgatcgttggcgtcggcaaacagtggtccggcgca cgcgctctggaggccttgctcacggtggcgggagagttgagaggtccaccgttacagttggacacaggccaacttctcaagat tgcaaaacgtggcggcgtgaccgcagtggaggcagtgcatgcatggcgcaatgcactgacgggtgccccctgaacctgact ccggaacaggtggtggcgattgcaagcaacatcggcggcaaacaggctctagagaccgtgcagcgcctgctgcccgtgctgt gccaggcccacggcctgacccccgagcaggtggtggccatcgccagcaataacggcggcaagcaggcgctagagaccgtgc agcgcctgctgcccgtgctgtgccaggcccacggcctgacccccgagcaggtggtggccatcgccagcaataacggcggcaag caggcgctagagaccgtgcagcgcctgctgcccgtgctgtgccaggcccacggcctgacccccgagcaggtggtggccatcgc cagcaatattggcggcaagcaggcgctagagaccgtgcagcgcctgctgcccgtgctgtgccaggcccacggcctgacccccg agcaggtggtggccatcgccagcaatggcggcggcaagcaggcgctagagaccgtgcagcgcctgctgcccgtgctgtgcca ggcccacggcctgacccccgagcaggtggtggccatcgccagcaatggcggcggcaagcaggcgctagagaccgtgcagcg cctgctgcccgtgctgtgccaggcccacggcctgacccccgagcaggtggtggccatcgccagcaatattggcggcaagcagg cgctagagaccgtgcagcgcctgctgcccgtgctgtgccaggcccacggcctgacccccgagcaggtggtggccatcgccagc aatggcggcggcaagcaggcgctagagaccgtgcagcgcctgctgcccgtgctgtgccaggcccacggcctgacccccgagc aggtggtggccatcgccagcaataacggcggcaagcaggcgctagagaccgtgcagcgcctgctgcccgtgctgtgccaggc ccacggcctgacccccgagcaggtggtggccatcgccagcaataacggcggcaagcaggcgctagagaccgtgcagcgcctg ctgcccgtgctgtgccaggcccacggcctgacccccgagcaggtggtggccatcgccagccacgacggcggcaagcaggcgct agagaccgtgcagcgcctgctgcccgtgctgtgccaggcccacggcctgacccccgagcaggtggtggccatcgccagcaatg gcggcggcaagcaggcgctagagaccgtgcagcgcctgctgcccgtgctgtgccaggcccacggcctgacccccgagcaggt ggtggccatcgccagcaataacggcggcaagcaggcgctagagaccgtgcagcgcctgctgcccgtgctgtgccaggcccac ggcctgacccccgagcaggtggtggccatcgccagccacgacggcggcaagcaggcgctagagaccgtgcagcgcctgctgc ccgtgctgtgccaggcccacggcctgacccccgagcaggtggtggccatcgccagcaatggcggcggcaagcaggcgctaga gaccgtgcagcgcctgctgcccgtgctgtgccaggcccacggcctgacccccgagcaggtggtggccatcgccagcaataacg gcggcaagcaggcgctagagaccgtgcagcgcctgctgcccgtgctgtgccaggcccacggcctgacccccgagcaggtggtg gccatcgccagcaatggcggcggcaagcaggcgctagagaccgtgcagcgcctgctgcccgtgctgtgccaggcccacggcct gacccccgagcaggtggtggccatcgccagcaatggcggcggcaagcaggcgctagagagcattgttgcccagctctccaga cctgatccggcgctagccgcgttgctagtcaaaagtgaactcgaggagaagaaatctgaacttcgtcataaattgaaatatgt gcctcatgaatatattgaattaattgaaattgccagaaatcccactcaggatagaattcttgaaatgaaggtaatggaatttt ttatgaaagtttatggatatagaggtgagcatttgggtggatcaaggaaaccggacggagcaatttatactgtcggatctcct attgattacggtgtgatcgtggatactaaagcttatagcggaggttataatctgccaattggccaagcagatgccatgcaaag ctatgtcgaagaaaatcaaacacgaaacaaacatatcaaccctaatgaatggtggaaagtctatccatcttctgtaacggaa tttaagtttttatttgtgagtggtcactttaaaggaaactacaaagctcagcttacacgattaaatcatatcactaattgtaatg gagctgttcttagtgtagaagagcttttaattggtggagaaatgattaaagccggcacattaaccttagaggaagtgagacg |

**[Table 5]**

| |
|---|
| TALEN right: |
| gatctacgcacgctcggctacagccagcagcaacaggagaagatcaaaccgaaggttcgttcgacagtggcgcagcaccac gaggcactggtcggccatgggtttacacacgcgcacatcgttgcgctcagccaacacccggcagcgttagggaccgtcgctgtc aagtatcaggacatgatcgcagcgttgccagaggcgacacacgaagcgatcgttggcgtcggcaaacagtggtccggcgca cgcgctctggaggccttgctcacggtggcgggagagttgagaggtccaccgttacagttggacacaggccaacttctcaagat tgcaaaacgtggcggcgtgaccgcagtggaggcagtgcatgcatggcgcaatgcactgacgggtgccccctgaacctgact ccggaacaggtggtggcgattgcaagcaacaacggcggcaaacaggctctagagaccgtgcagcgcctgctgcccgtgctgt gccaggcccacggcctgacccccgagcaggtggtggccatcgccagcaatattggcggcaagcaggcgctagagaccgtgca gcgcctgctgcccgtgctgtgccaggcccacggcctgacccccgagcaggtggtggccatcgccagccacgacggcggcaagc aggcgctagagaccgtgcagcgcctgctgcccgtgctgtgccaggcccacggcctgacccccgagcaggtggtggccatcgcc agcaacaacggcggcaagcaggcgctagagaccgtgcagcgcctgctgcccgtgctgtgccaggcccacggcctgacccccg agcaggtggtggccatcgccagcaatggcggcggcaagcaggcgctagagaccgtgcagcgcctgctgcccgtgctgtgcca ggcccacggcctgacccccgagcaggtggtggccatcgccagcaatggcggcggcaagcaggcgctagagaccgtgcagcg cctgctgcccgtgctgtgccaggcccacggcctgacccccgagcaggtggtggccatcgccagccacgacggcggcaagcagg cgctagagaccgtgcagcgcctgctgcccgtgctgtgccaggcccacggcctgacccccgagcaggtggtggccatcgccagc aatggcggcggcaagcaggcgctagagaccgtgcagcgcctgctgcccgtgctgtgccaggcccacggcctgacccccgagc aggtggtggccatcgccagcaatattggcggcaagcaggcgctagagaccgtgcagcgcctgctgcccgtgctgtgccaggcc cacggcctgacccccgagcaggtggtggccatcgccagcaacaacggcggcaagcaggcgctagagaccgtgcagcgcctgc tgcccgtgctgtgccaggcccacggcctgacccccgagcaggtggtggccatcgccagcaatggcggcggcaagcaggcgcta gagaccgtgcagcgcctgctgcccgtgctgtgccaggcccacggcctgacccccgagcaggtggtggccatcgccagcaatgg cggcggcaagcaggcgctagagaccgtgcagcgcctgctgcccgtgctgtgccaggcccacggcctgacccccgagcaggtg gtggccatcgccagcaacaacggcggcaagcaggcgctagagaccgtgcagcgcctgctgcccgtgctgtgccaggcccacg gcctgacccccgagcaggtggtggccatcgccagccacgacggcggcaagcaggcgctagagaccgtgcagcgcctgctgcc cgtgctgtgccaggcccacggcctgacccccgagcaggtggtggccatcgccagcaatggcggcggcaagcaggcgctagag accgtgcagcgcctgctgcccgtgctgtgccaggcccacggcctgacccccgagcaggtggtggccatcgccagccacgacggc ggcaagcaggcgctagagaccgtgcagcgcctgctgcccgtgctgtgccaggcccacggcctgacccccgagcaggtggtggc catcgccagccacgacggcggcaagcaggcgctagagaccgtgcagcgcctgctgcccgtgctgtgccaggcccacggcctga cccccgagcaggtggtggccatcgccagcaatggcggcggcaagcaggcgctagagaccgtgcagcgcctgctgcccgtgct gtgccaggcccacggcctgacccccgagcaggtggtggccatcgccagcaacaacggcggcaagcaggcgctagagaccgt gcagcgcctgctgcccgtgctgtgccaggcccacggcctgacccccgagcaggtggtggccatcgccagcaatggcggcggca agcaggcgctagagagcattgttgcccagctctccagacctgatccggcgctagccgcgttgctagtcaaaagtgaactcgag gagaagaaatctgaacttcgtcataaattgaaatatgtgcctcatgaatatattgaattaattgaaattgccagaaatcccac tcaggatagaattcttgaaatgaaggtaatggaatttttttatgaaagtttatggatatagaggtgagcatttgggtggatca aggaaaccggacggagcaatttatactgtcggatctcctattgattacggtgtgatcgtggatactaaagcttatagcggagg ttataatctgccaattggccaagcacgagaaatgcaacgatatgtcgaagaaaatcaaacacgaaacaaacatatcaaccc |

### (4. Targeting of selection cassette on AAVS1 region of chromosome 19 and selection of one allele target clone)

By inserting arms of the AAVS1 region of chromosome 19 into a basic vector, a vector for targeting AAVS1 of chromosome 19 was constructed. Also, using 8 µg of DNA (AAVS1-ZFN left 2 µg, AAVS1-ZFN right 2 µg, and targeting vector for AAVS1 4 µg), one million BLM^{tet/tet} hiPSC in a 100 µl tip were transfected by electroporation (Neon transfection platform; Invitrogen) in the conditions of 1100 V, 30 msec, and 1 pulse. As the sequence of AAVS1-ZFN, the one reported in Hockemeyer D, Soldner F, Beard C, Gao Q, Mitalipova M, Dekelver RC, Jaenisch R, et al. Efficient targeting of expressed and silent genes in human ESCs and iPSCs using zinc-finger nucleases. Nature biotechnology. 2009;27(9):851-857. was used.

The cells subjected to transfection were plated in a hESC culture medium containing 10 mM Y-27632 but not containing G418 on a Synthemax^{(R)} II-SC-coated tissue culture plate. After two days from the transfection, selection was started with 100 µg/ml of G418. Target clones were selected first by PCR using a primer AAVS1-LF2 and a primer Puro-L2, and then one allele target clones (hiPSC-BLM ^{tet/tet}AAVS1 ^{cNP/+}) were selected by PCR using primers AAVS1-C-F, AAVS1-C-R, and Puro-L2.

**[Table 6]**

| | |
|---|---|
| AAVS1-LF2 | GATGCAGGGGAACGGGGCTCAGTCTGAAGA |
| Puro-L2 | GCGAGGCGCACCGTGGGCTTGTACTCGGTC |

**[Table 7]**

| | |
|---|---|
| AAVS1-C-F | ACTAGGAAGGAGGAGGCCTAAGGATGGGGC |
| AAVS1-C-R | GCTCTTCCAGCCCCCTGTCATGGCATCTTC |
| Puro-L2 | GCGAGGCGCACCGTGGGCTTGTACTCGGTC |

### (5. Heterozygouse allele specific introduction of DSB in chromosome 19 and selection of homozygous cells)

One million BLM^{tet/tet} AAVS1^{cNP/+} hiPSC cells were transfected with 6.5 to 8.0 µg of CRISPR/Cas 9 (pX330 containing various gRNA sequences) and 3 µg of iCre recombinase plasmid by electroporation using a Neon^{(R)} Transfection System in the conditions of 1300 V, 20 msec, and 1 pulse. As the gRNA sequence, the one shown in the later-described Table 12 was used.

The cells subjected to transfection were plated in a hESC culture medium containing 10 mM Y-27632 but not containing puromycin and G418 on a Synthemax^{(R)} II-SC-coated tissue culture plate. After two days from the transfection, selection was started with 0.5 µg/ml puromycin. After four days from the transfection, the selection condition was changed to 1.0 µg/ml of puromycin and 100 µg/ml of G418 (for neomycin selection). After six days, the selection condition was changed to 1.0 µg/ml of puromycin and 200 µg/ml of G418. In this manner, homozygous cells hiPSC-BLM ^{tet/tet}AAVS1 ^{cNP/+}Dox(+)9M-CRISPR(+) were obtained.

### [Example] Production of homozygous cells by heterozygouse allele specific DNA dNA double strand break using BLM protein inhibitor

In one million cells cultured for 12 hours in the presence of BLM protein inhibitor ML216 (10.0 µM, 12.5 µM, and 14.0 µM), CRISPR/Cas9 that is designed to heterozygouse allele specifically introduce DSB was introduced without using Tet-off, and by conducting double drug selection with neomycin and puromycin, cell strains of site-specific homozygotes respectively aiming at the AAVS1 region of chromosome 19 (9 Mb from centromere (35M region), 14 Mb from centromere (40M region), and 19 Mb-I, 19 Mb-II from centromere (45M-1 region, 45M-2 region) as target sites were acquired. The 19 Mb-I, 19 Mb-II from centromere (45M-1 region, 45M-2 region) are different regions in 45M region. Further, in Example, also a cell strain of a site-specific homozygote respectively aiming at the HLA region of chromosome 6 as a target site was acquired. In the double drug selection cassette, two kinds of genes (neomycin and puromycin) enabling drug selection are arranged in directions opposite to each other, and oppositely-directed loxPs exist outside the two kinds of genes. The double drug selection cassette becomes two copies by changing into a site-specific homozygote, and becomes resistant to the two kinds of drugs by the action of a Cre recombinase.

### (1. Preparation of selection cassette for detecting site-specific homozygote)

In the same manner as Reference example, a selection plasmid was prepared in the following manner.

First, bGH (bovine growth hormone) polyA sequence necessary for expression was amplified in the following conditions using the following template and primers.
Template:
   PGK-L21-NPT-L21-pA (A homozygous mutant embryonic stem cell bank applicable for phenotype-driven genetic screening. Nature Methods 8, 1071-1077 (2011))
Primers:
   *KpnI-bGHpA-puro-up-up:TAGTGGGCGCGCCGGTACCTTATCGAGTCC
   *Gly -puro-low:GGCTTGTACTCGGTCATGGT
      GGATCCAATAACTTCGTATAATGT
PCR conditions:
   94°C for 2 min, (98°C for 10 sec, 68°C for 1 min) × 35 cycles, and 68°C for 7 min

The puromycin gene was amplified in the following conditions using the following template and primers.
Template:
   pPGKpuro-F2F
Primers:
   *Gly -puro- up:GACCGAGTACAAGCC CACGGTGCGCCTCGC
   *puro-up:TAA CTCGAGTCATTA GGCACCGGGCTTGCGGGTCATGCAC
PCR conditions:
   94°C for 2 min, (98°C for 10 sec, 68°C for 1 min) x 35 cycles, and 68°C for 7 min

The neomycin gene was amplified in the following conditions using the following template and primers.
Template:
   pMulti-hEF1a-LP1-NPT-LP1-SLRarm
Primers:
   neo-low:TAATGACTCGAGTTAGAAGAACTCGTCAAGAAGGCGATAG
   XhoI-tTA-up:TCGATTAGTTCTCGAGCTTTTGGAGTACGT
PCR conditions:
   94°C for 2 min, (98°C for 10 sec, 68°C for 1.5 min) x 35 cycles, and 68°C for 7 min

The three fragments obtained in the manner as described above were introduced by In Fusion method into a basic vector having a hEF1α promoter and a targeting arm cloning site of PmeI/AscI/SfiI and inserted therein.

### (2. Targeting of selection cassette)

### (2-1. Targeting of selection cassette on AAVS1 region of chromosome 19)

By inserting arms of the AAVS1 region of chromosome 19 into a basic vector, a vector for targeting AAVS1 of chromosome 19 was constructed. Also, using 8 µg of DNA (AAVS1-ZFN left 2 µg, AAVS1-ZFN right 2 µg, and targeting vector for AAVS1 4 µg), one million human iPS cells were transfected by using the Neon^{(R)} Transfection System in the conditions of 1100 V, 30 msec, and 1 pulse. As the sequence of AAVS1-ZFN, the one reported in Hockemeyer D, Soldner F, Beard C, Gao Q, Mitalipova M, Dekelver RC, Jaenisch R, et al. Efficient targeting of expressed and silent genes in human ESCs and iPSCs using zinc-finger nucleases. Nature biotechnology. 2009;27(9):851-857. was used.

By conducting PCR (94°C for 2 min, (98°C for 10 sec, 68°C for 30 sec) x 35 cycles, and 68°C for 7 min) using a primer AAVS1-LF2 on the outer side than the arm of the targeting vector (chromosome 19 + 55114902-55114931) and a primer Puro-L2 inside the selection cassette (on puromycin inside the selection cassette), insertion of the selection cassette was confirmed.

**[Table 8]**

| | |
|---|---|
| AAVS1-LF2 | GATGCAGGGGAACGGGGCTCAGTCTGAAGA |
| Puro-L2 | GCGAGGCGCACCGTGGGCTTGTACTCGGTC |

In order to make the selection cassette removable after homologous recombination by crossover, a vector in which the aforementioned selection cassette is introduced into a vector having a recognition sequence of transposon was prepared.

### (2-2. Targeting of selection cassette on HLA region of chromosome 6)

In a vector having a recognition sequence of transposon (PiggyBac transposon vector), a selection cassette, and an insulator sequence for protecting the inserted exogenous DNA (selection cassette) from inactivation such as methylation, on both sides of the selection cassette were incorporated. In a targeting arm cloning site of the vector having the selection cassette and the insulator sequence incorporated therein, a PCR amplified product of a sequence on the telomere side of the HLA region of chromosome 6 was incorporated to give a targeting vector.

### - Introduction of insulator sequence into PiggyBac transposon vector

An insulator sequence (tgcttgtccttccttcctgtaacacagccattaaaccaggagcatcgcccttccccggccctcaggtaagaggaccaaatacc gtagccgtttccaatttcagtcctttagcgccacctggtgctaactactctatcacgcttttatccaataactacctttgtaaatttcctt tcaaaagttctggccgggcgcggtggctcacgcttgtaatcccagcactttgtgaggggtcaggagttc; chromosome 7 +39519983-39520225) was amplified with a primer PB5'-HindIII-ins and a primer ins-HindIII-PB5' on 5' end, and amplified with a primer bghpA-ins and a primer ins-SpeI-PB3' on 3' end, and incorporated into the PiggyBac transposon vector (#28 pPB-hEF1-puro-bghpA-h5 F-insurator-2).

**[Table 9]**

| | |
|---|---|
| PB5' -HindIII-ins | TATAGATATCAAGCTTTGCTTGTCCTTCCTTCCTGTAACAC |
| ins-HindIII-PB5 | CGGTATCGATAAGCTTGAACTCCTGACCCCTCACAAAGTGC |
| bghpA-ins | GGGTCGACATACTAGATGCTTGTCCTTCCTTCCTGTAACAC |
| ins-speI-PB3' | AAAACTTTTAACTAGTGAACTCCTGACCCCTCACAAAGTGC |

### - Insertion of targeting arm

In the AscI site, an arm amplified with the following primer chr6-Asc1-up and primer chr6-Asc1-low was inserted. In the PacI site, an arm amplified with the following primer chr6-Pac1-up and primer chr6-Pac1-low was inserted. PCR conditions were 94°C for 2 min (98°C for 10 sec, 68°C for 1 min) x 35 cycles, and 68°C for 7 min.

**[Table 10]**

| | |
|---|---|
| chr6-Asc1-up | GGGCGAATTGGGGCGCGCCGGACAAAGCCACTGAAGGAGAGTAGG |
| chr6-Asc1-low | TAATCTAGAATGGCGCGCCGTGGCGGCTATTTTCTCTCCTACAGC |
| chr6-Pac1-up | GTATGCGGCCGCTTAATTAATTAAAGGACTGCAGGAAAAACAGGGTC |
| chr6-Pac1-low | ACAAAAGCTCGATTAATTAATTGGACTGCCACCTTGCAGAGGGAGC |

One million human iPS cells were transfected with 8 µg of CRISPR/Cas9 that recognizes the part (agaaaatagccgccacttaaagg) where the aforementioned arms join on the genome, together with 3 µg of the targeting vector by using Neon^{(R)} Transfection System in the conditions of 1200 V, 30 msec, and 1 pulse.

In order to confirm that the selection cassette is inserted only in one of the allele, and no deletion or the like occurs, PCR was conducted simultaneously with the following three primers (chr6-TG-low, chr6-TG-up, and hEF1a-TG). PCR conditions were 94°C for 2 min, (98°C for 10 sec, 68°C for 1.5 min) x 35 cycles, and 68°C for 7 min.

**[Table 11]**

| | |
|---|---|
| chr6-TG-low | CATGCAGCAAAATCCTTTATATTTTACATC |
| chr6-TG-up | GAGCTGGAGCTACTGGTAATGACAAGGTC |
| hEF1a-TG | GGATCAAGAATCACGTACTGCAGCCAGGTG |

The PCR products were subjected to electrophoresis, and a clone in which both a band of 1320 bp (with insertion of selection cassette) and a band of 570 bp (wild-type without insertion of selection cassette) were detected was acquired as the clone in which the cassette is inserted only in one allele.

### (3. Construction of CRISPR/Cas9 system for heterozygouse allele specific DNA double strand break introduction)

In order to conduct DNA double strand break (DSB) by a CRISPR/Cas9 system aiming at the AAVS1 region (9 Mb from centromere (35M region), 14 Mb from centromere (40M region), 19 Mb-I, 19 Mb-II from centromere (45M-1 region, 45M-2 region) of chromosome 19, and the HLA region (29M region) of chromosome 6 as target sites, a gRNA having SNP in PAM (NGG) located on the 3' side of the target site was prepared, and a CRISPR/Cas9 system was constructed so that DSB is selectively introduced only in one allele. SNP information (SNP type indicates an SNP type at "G" of the PAM sequence NGG. At which G the SNP type is located is clearly shown by SNP_ID in Table 12.), position of target site on chromosome, and gRNA targeting sequence are shown in the following table.

**[Table 12]**

| SNP_ID | SNP type | chromosome position | gRNA targeting sequence |
|---|---|---|---|
| rs2965273 | A/G | Chr19_4512783-34512803(35M) | CCTGGTGTGGGGGCCCTGCG (SEQ ID NO: 1) |
| rsl7656487 | C/T | Chr19_40498170-40498189(40M) | GTCAGGGATGGGATTAGGGA (SEQ ID NO: 2) |
| rs12977652 | A/G | Chr19_4450071-44500590(45M-1) | CCCAGAAGCCTCCGCGGCGC (SEQ ID NO: 3) |
| rs1727740 | C/T | Chr19_44636861-44536880(45M-2) | ATTGTTATATTGGTGAGGGG (SEQ ID NO: 4) |
| rs2246618 | C/T | Chr6_2810497-2810516(29M) | ATTGCTTTGATGCTGGGTCA (SEQ ID NO: 5) |

For example, in order to insert a gRNA targeting sequence of 35M region for breaking one allele into pX330, a 100 µM solution of oligo DNA having the following sequence in TE was prepared, and after adding 30 µl of ion exchange water to 10 µl of the solution, the solution was heated at 100°C, and then left to stand until the solution cools to room temperature to allow annealing. Thereafter, the annealed oligo DNA was cloned into the restriction enzyme BbsI site of the pX330 vector to construct the CRISPR/Cas9.
- Oligo DNA for 35M region of chromosome 19:
   CACCGCCTGGTGTGGGGGCCCTGCG
   AACCGCAGGGCCCCCACACCAGGC

Also in the case of setting the following regions as target sites, the same procedure was conducted except for using the following oligo DNA.
- Oligo DNA for 40M region of chromosome 19:
   CACCGGTCAGGGATGGGATTAGGGA
   AAACTCCCTAATCCCATCCCTGACC
- Oligo DNA for 45M-1 region of chromosome 19:
   CACCGCCCAGAAGCCTCCGCGGCGC
   AAACGCGCCGCGGAGGCTTCTGGGC
- Oligo DNA for 45M-2 region of chromosome 19:
   CACCGATTGTTATATTGGTGAGGGG
   AAACCCCCTCACCAATATAACAATC
- Oligo DNA for HLA region of chromosome 6
   CACCGATTGCTTTGATGCTGGGTCA
   AAACTGACCCAGCATCAAAGCAATC

### (4. Cell culture)

Human induced pluriopotent stem cell (hiPSC) line (Igawa K, Kokubu C, Yusa K, Horie K, Yoshimura Y, Yamauchi K, Suemori H, Yokozeki H, Toyoda M, Kiyokawa N, Okita H, Miyagawa Y, Akutsu H, Umezawa A, Katayama I, Takeda J. Removal of reprogramming transgenes improves the tissue reconstitution potential of keratinocytes generated from human induced pluripotent stem cells. Stem Cells Transl Med. 2014 Sep; 3(9): 992-1001.) was cultured in a hESC culture medium on a Synthemax^{(R)} II-SC (Corning) coated tissue culture plate, and subcultured by using Accutase^{(R)}, and plated using an Rho kinase inhibitor Y-27632 (10 µM, LC laboratories).

### (5. Use amount and use method of Bloom's syndrome protein inhibitor (ML216))

ML216 in a concentration of 10.0 µM, 12.5 µM, or 14.0 µM was caused to act on cells during the period from 12 hours before introduction (transfection) of a CRISPR/Cas9 that induces crossover, and a Cre recombinase that actuates the selection cassette to 48 hours after the introduction. Specifically, in the above-described cell culture, ML216 dissolved in DMSO was contained in the hESC culture medium in the above concentration.

### (6. Heterozygouse allele specific introduction of DSB and selection of homozygous cells)

### (6-1. Heterozygouse allele specific introduction of DSB in chromosome 19 and selection of homozygous cells)

One million human induced pluriopotent stem cells (human iPS cells (hiPSCs)) were transfected with 8 µg of CRISPR/Cas 9 (pX330 containing gRNA sequence according to Table 12) and 3 µg of iCre recombinase plasmid by electroporation using a Neon^{(R)} Transfection System in the conditions of 1300 V, 20 msec, and 1 pulse.

The cells subjected to transfection were plated in a hESC culture medium containing 10 mM Y-27632 but not containing puromycin and G418 on a Synthemax^{(R)} II-SC-coated tissue culture plate. After two days from the transfection, selection was started with 0.5 µg/ml puromycin. After four days from the transfection, the selection condition was changed to 1.0 µg/ml of puromycin and 100 µg/ml of G418 (for neomycin selection). After six days, the selection condition was changed to 1.0 µg/ml of puromycin and 200 µg/ml of G418. Thus, homozygous cells hiPSC-AAVSl ^{cNP/+}ML216(+)9M-CRISPR(+) were obtained.

### (6-2. Heterozygouse allele specific introduction of DSB in chromosome 6 and selection of homozygous cells)

One million human induced pluriopotent stem cells (human iPS cells (hiPSCs)) were transfected with 8 µg of CRISPR/Cas 9 (pX330 containing gRNA sequence according to Table 12) and 3 µg of iCre recombinase plasmid by electroporation using a Neon^{(R)} Transfection System in the conditions of 1300 V, 20 msec, and 1 pulse.

For the cells subjected to transfection, selection with 0.2 µg/ml of puromycin was started after two days from the transfection. After four days from the transfection, the selection condition was changed to 0.25 µg/ml of puromycin and 25 µg/ml of G418. After six days, the selection condition was changed to 0.33 µg/ml of puromycin and 50 µg/ml of G418. In this manner, homozygous cells hiPSC-telHLA ^{cNP/+}ML216(+)HLA I-III-CRISPR(+) were obtained.

### [Result 1 by Reference example and Examples]

### (1. Treatment of homozygous cells)

As will be described below, for the homozygous cells obtained in Reference example and Examples (hiPSC-BLM ^{tet/tet}AAVS1 ^{cNP/+}Dox(+)9M-CRISPR(+) obtained in Reference example, and hiPSC-AAVS1^{cNP/+}ML216(+)9M-CRISPR(+) and hiPSC-telHLA^{cNP/+}ML216(+)HLA I-III-CRISPR(+) obtained in Examples), RNA extraction and cDNA synthesis, DNA extraction, screening of homozygous cells (genotyping), determination of crossover site, determination of HLA genotype (in the cases of Examples), and flow cytometry were conducted.

### (1-1. RNA extraction and cDNA synthesis)

The total RNA sample was prepared from cells using an RNeasy Plus Micfro Kit (QIAGEN). A single-stranded cDNA was synthesized from 800 ng of total RNA using SuperScriptIII (Invitrogen), at 50°C for 60 minutes with a random hexamer primer.

### (1-2. DNA extraction)

Cells were incubated at 55°C overnight in a lysis butter (10 mM TrisHCl, pH 8.0, 1 mM EDTA, X1SSC, 1% SDS, and 200 µg/ml protease K). DNA was extracted from hiPSC using a DNA extraction kit DNeasy^{(R)}(Qiagen).

### (1-3. Screening of homozygous cells (genotyping))

### (1-3-1. Screening of homozygous cells in chromosome 19)

DNA extracted from a hiPSC colony after selection was subjected to competitive PCR using the following primers (AAVS1-C-F, AAVS1-C-R, and Puro-L2). PCR conditions were 94°C for 2 min, (98°C for 10 sec, 68°C for 1.5 min) x 35 cycles, and 72°C for 7 min.

**[Table 13]**

| | |
|---|---|
| AAVS1-C-F | ACTAGGAAGGAGGAGGCCTAAGGATGGGGC |
| AAVS1-C-R | GCTCTTCCAGCCCCCTGTCATGGCATCTTC |
| Puro-L2 | GCGAGGCGCACCGTGGGCTTGTACTCGGTC |

### (1-3-2. Screening of homozygous cells in chromosome 6)

DNA extracted from hiPSC colonies after selection was subjected to competitive PCR using the following primers (chr6-TG-check-up-outside, chr6-TG-check-low-outside, and Puro-L2). PCR conditions were 94°C for 2 min (98°C for 10 sec, 68°C for 2 min 10 sec) x 35 cycles, and 72°C for 7 min.

**[Table 14]**

| | |
|---|---|
| chr6-TG-cheek-up-outside | GAGCTGGAGCTACTGGTAATGACAAGGTC |
| chr6-TG-check-low-outside | ATGATTGCTCAATGTAGGACCTGGCCTCAC |
| Puro-L2 | GCGAGGCGCACCGTGGGCTTGTACTCGGTC |

### (1-4. Determination of crossover site of chromosome 19 and chromosome 6)

First, genomic DNA was extracted from cells before occurrence of chromosomal crossover, and a SNP having different sequences between two chromosomes (hetero state) was identified. Also, after occurrence of crossover reaction, genomic DNA was extracted from a positive clone identified by competitive PCR, and the SNP site was analyzed to analyze whether the sequence had changed to one type (homo state). As a result of the analysis, the middle position between the SNP site in hetero state and the SNP site in homo state was determined as a crossover site (CO).

### (1-5. Determination of HLA genotype)

First, target DNA was amplified by using group-specific primers (LABType SSO HLA A Locus, LABType SSO HLA B Locus, LABType SSO HLA C Locus, and LABType SSO HLA DRB1). For detection using R-phycoerythrin-conjugated streptavidin, the PCR product was biotinized. The PCR product was denatured, and allowed to re-hybridize with a cDNA probe bound to fluorescent beads (One Lamda). Fluorescent intensity of phycoerythrin on each bead was identified by a LABScan 100(Luminex ^{(R)} 100) flow analyzer. By comparing the patterns of positive and negative beads ID with a LABType SSO worksheet or information of HLA Fusion 4.1 HotFix 2, a HLA allele or an allele group in the sample was determined.

### (1-6. Flow cytometry)

A suspension of hiPSC was washed with a phosphate buffered saline containing 1%(wt/vol) bovine serum albumin and 0.05%(v/v) sodium azide, and then incubated for 30 minutes on ice together with appropriate antibodies. The sample was analyzed by using BD FACSCanto II (BD Biosciences). The data was analyzed by using FlowJo software (Tomy Digital Biology). The antibodies used in the flow cytometry were FITC-conjugated anti HLA-A2 antibody (BioLegend, 343303), anti HLA-A32/25 monoclonal IgM antibody (One Lamda, 0136HA), and Alexa Fluor 647-conjugated goat anti-mouse IgM (Abcam ab150123).

### (2. Results)

### (2-1. Screening result of homozygous cells (genotyping result))

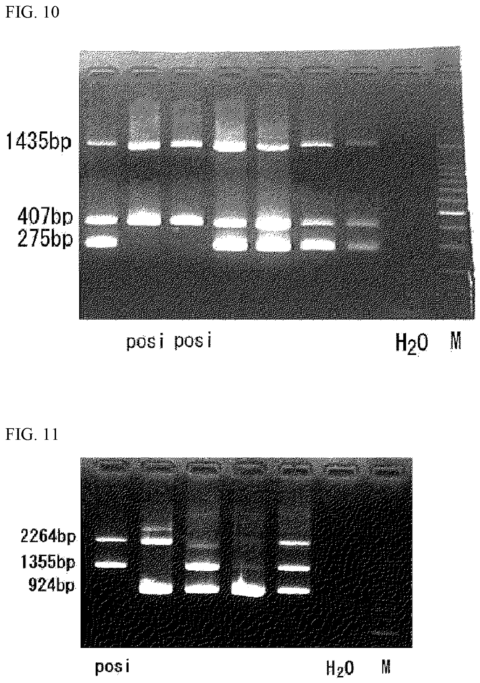

The result of competitive PCR conducted for screening homozygous cells in chromosome 19 in Reference example using Tet off is shown in Fig. 9. The wild-type allele was detected at 275 bp, the neomycin selective allele was detected at 407 bp, and the puromycin selective allele was detected at 1435 bp. A clone (posi) in which a wild-type band was not detected, but merely bands of 407 bp and 1435 bp that were positive for both of the neomycin selectivity and puromycin selectivity were detected was acquired as objective homozygous cells. A clone in which bands were detected at 275 bp as well as at 407 bp and 1435 bp is a background clone that survived by making originally two chromosomes into four chromosomes, or by amplifying the selection cassette to a plurality of the selection cassettes on the same chromosome. The result of competitive PCR conducted for screening homozygous cells in chromosome 19 in Example using ML216 is shown in Fig. 10. The result of Fig. 10 was similar to that of Fig. 9, and a clone in which a wild-type band was not detected, but merely bands that were positive for both of the neomycin selectivity and puromycin selectivity were detected was acquired as objective homozygous cells.

The result of competitive PCR conducted for screening homozygous cells in chromosome 6 in Example using ML216 is shown in Fig. 11. The wild-type allele was detected at 924 bp, the neomycin selective allele was detected at 1355 bp, and the puromycin selective allele was detected at 2264 bp. A clone in which a wild-type band was not detected, but merely bands of 1355 bp and 2264 bp that were positive for both of the neomycin selectivity and puromycin selectivity (posi) were detected was acquired as objective homozygous cells.

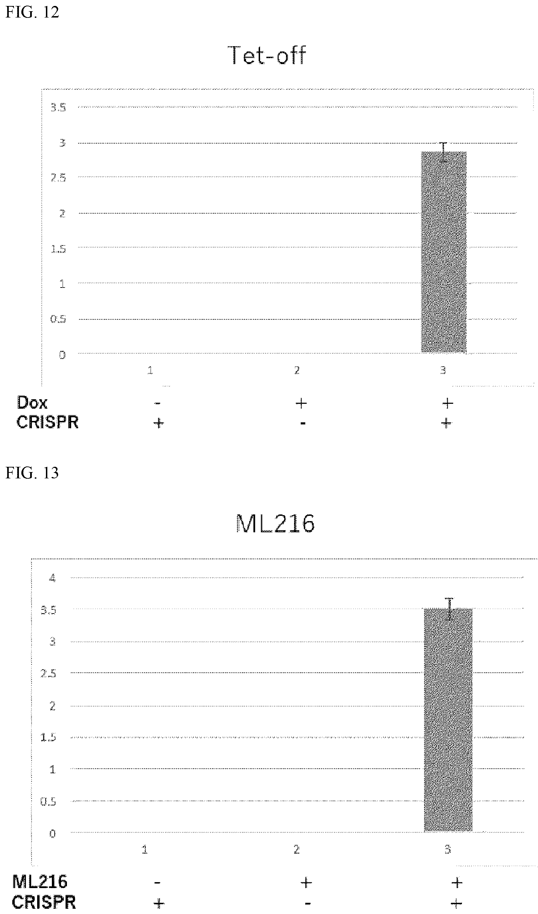

The crossover efficiency of Reference example using Tet off (sample of 3 in the drawing) is shown in Fig. 12 together with the crossover efficiencies (examined seven times for each sample) of the cases (samples 1, 2 in the drawing) where various conditions (presence or absence of Dox, and presence or absence of CRISPR) are changed in the Reference example. Also, the crossover efficiency of Example using ML216 (sample of 3 in the drawing) is shown in Fig. 13 together with the crossover efficiencies (examined four times for each sample) of the cases (samples 1, 2 in the drawing) where various conditions (presence or absence of ML216, and presence or absence of CRISPR) are changed in the Example.

In any of Reference example and Examples, similarly, it was possible to obtain objective homozygous cells by crossover in each of chromosome 19 and chromosome 6. Comparison between Reference example and Examples revealed that homozygous cells could be obtained with higher frequency in Examples. Further, in Examples, the objective homozygous cells could be obtained in all the cases using ML216 in concentrations of 10.0 µM, 12.5 µM, and 14.0 µM, and it was confirmed that homozygous cells could be obtained with the highest frequency (Fig. 13) when ML216 was used in a concentration of 12.5 µM.

### (2-2. Analysis result of crossover site)

### (2-2-1. Analysis result of crossover site in chromosome 19)

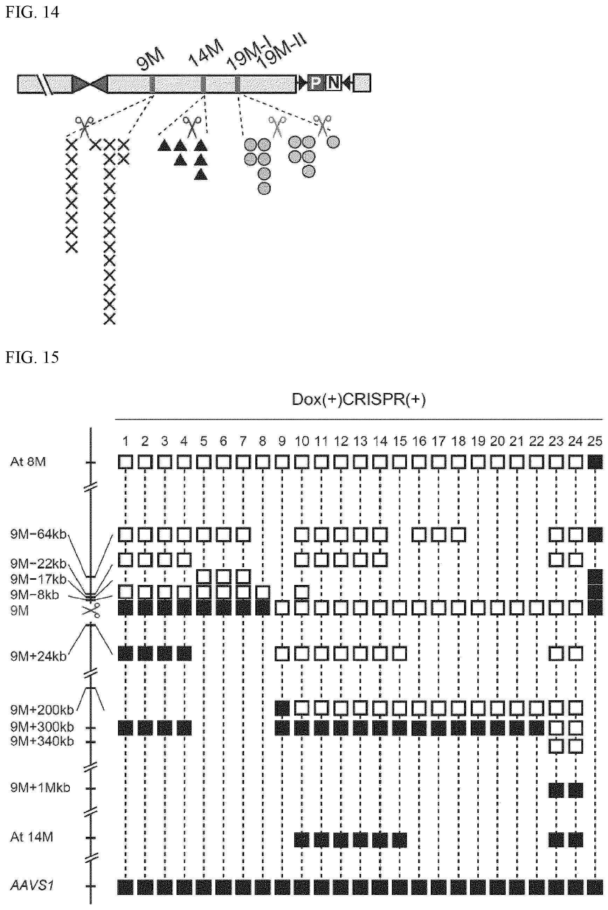

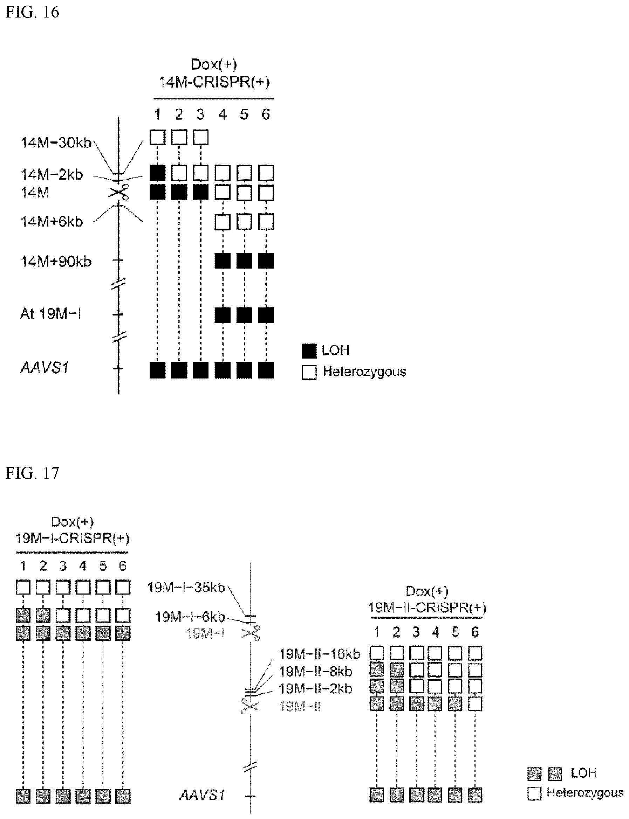

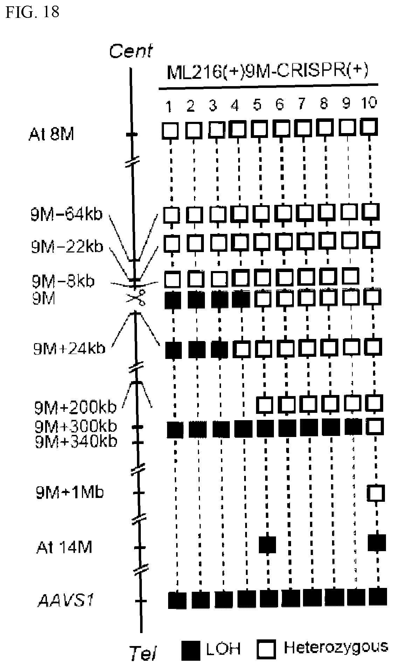

Fig. 14 shows distribution of crossover sites in chromosome 19 (Reference example). In Fig. 14, each point denoted by × indicates a clone in which DSB is generated in 9 Mb (35M region) from centromere of chromosome 19; each point denoted by ▲ indicates a clone in which DSB is generated in 14 Mb (40M region) from centromere; each point denoted by ○ indicates a clone in which DSB is generated in 19Mb-I, 19Mb-II (45M-1, 45M-2 regions) from centromere. Fig. 15 shows distribution of crossover sites at 9 Mb (35M region) from centromere of chromosome 19 (Reference example). Fig. 16 shows distribution of crossover sites at 14 Mb (40M region) from centromere of chromosome 19 (Reference example). Fig. 17 shows distribution of crossover sites at 19Mb-I, 19Mb-II (45M-1, 45M-2) from centromere of chromosome 19 (Reference example). Fig. 18 shows distribution of crossover sites at 9 Mb (35M region) from centromere of chromosome 19 under suppression of BLM protein by ML216.

As shown in Fig. 14 to Fig. 18, in any of Reference example and Examples, crossover occurred in the neighborhood of introduced DSB, and as shown in Fig. 15 and Fig. 18, the sites at which crossover occurred were well matched between Reference example and Examples.

### (2-2-2. Analysis result of crossover site in chromosome 6)

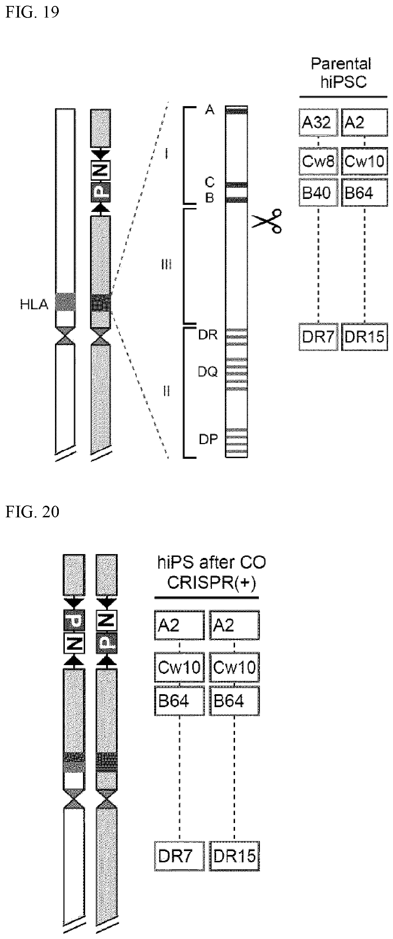

Fig. 19 schematically shows the short arm of chromosome 6 before crossover. After targeting of the double selection cassette, a heterozygouse allele specific DSB was introduced between HLA class I and class III. The HLA genotype of the parental cell is shown in the right panel. Fig. 20 shows a schematic view of the short arm of chromosome 6 after crossover. The HLA genotype after crossover is shown in the right panel. Also in chromosome 6, it was confirmed that crossover occurred in the neighborhood of introduced DSB.

### (2-3. Result of flow cytometry)

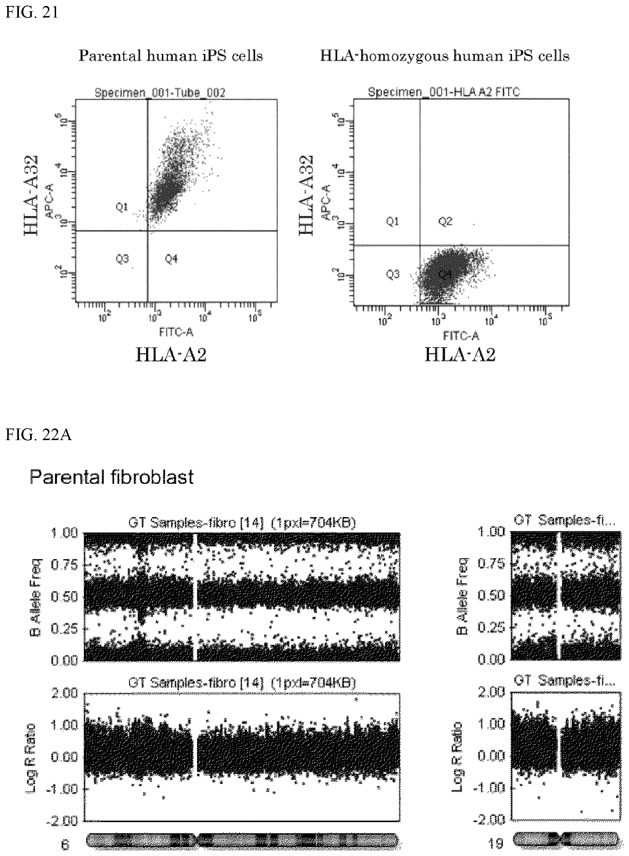

Fig. 21 shows a flow cytometry profile of HLA-A haplotype in hiPSC (Example). As shown in Fig. 21, the flow cytometry demonstrated that the haplotype of the HLA-homozygous human iPS cells having LOH in this region as a result of occurrence of crossover using the Bloom's syndrome protein inhibitor (ML216) was homo of only HLA-A2, while the haplotype of the parental human iPS cells before occurrence of crossover was hetero of A2 and A32 in the HLA-A region.

### (2-4. Conclusion)

The results of genotyping and flow cytometry demonstrated that LOH associated with chromosomal crossover occurs in an expected position of the homozygous cells.

### [Result 2 by Reference example and Examples]

### (1. Treatment of cells)

For analyzing the number of SNP copies, genomic DNA was isolated from parental fibroblast cells (cells from which hiPSCs are derived), hiPSCs, hiPSC-BLM^{tet/tet}AAVS1^{cNP/+} obtained in item (4) of Reference example, and homozygous cells obtained in Reference example and Examples (hiPSC-BLM^{tet/tet}AAVS1^{cNP/+}Dox(+)9M-CRISPR(+) obtained in Reference example, and hiPSC-AAVS1^{cNP/+}ML216(+)9M-CRISPR(+) and hiPSC-telHLA^{cNP/+}ML216(+)HLA I-III-CRISPR(+) obtained in Examples), and hybridized with Infinium Omni5-4 v1.2 BeadChip(Illumina). A single nucleotide polymorphism (SNP) array analysis was conducted using GenomeStudio (Illumina).

### (2. Results)

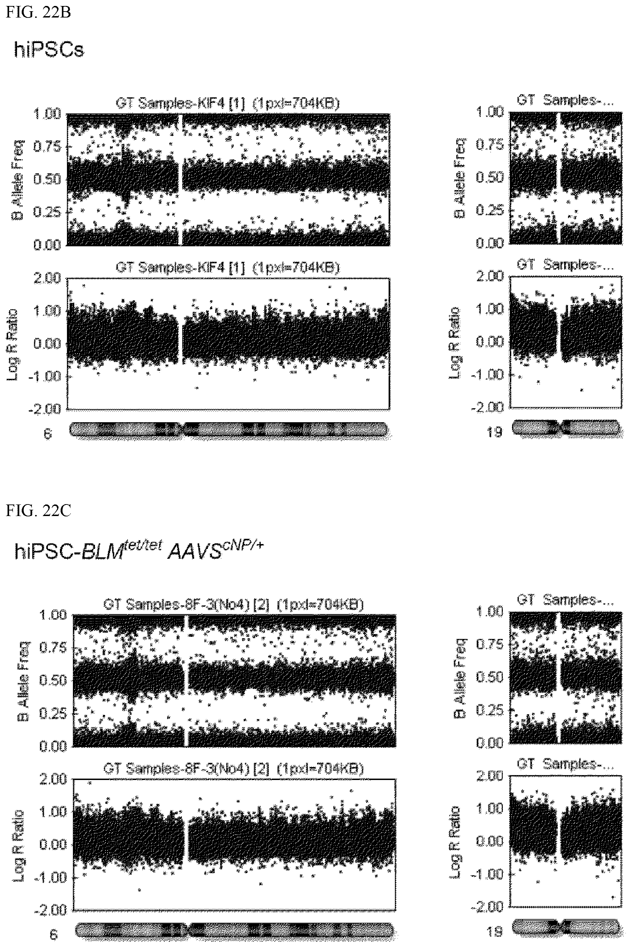

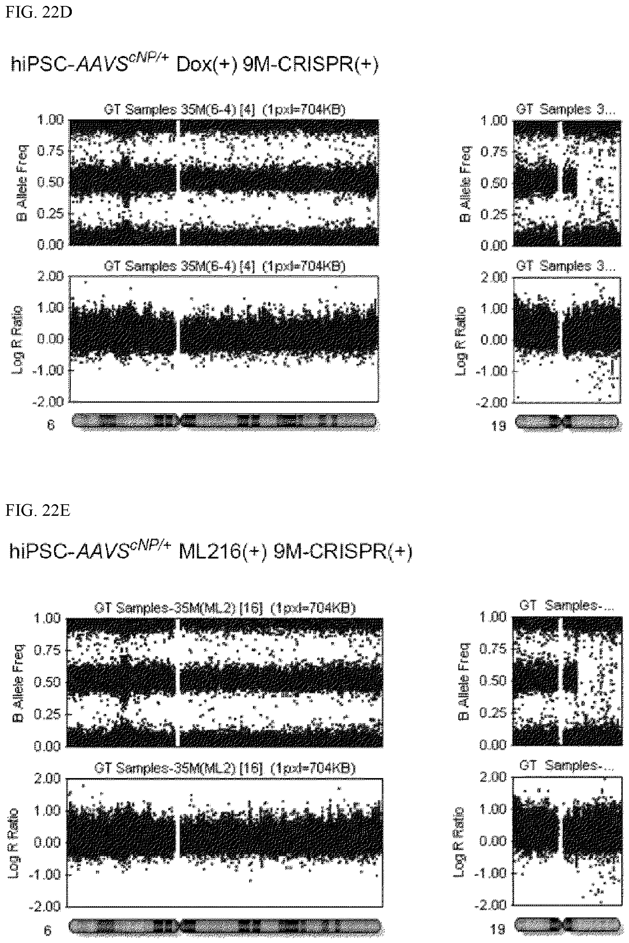

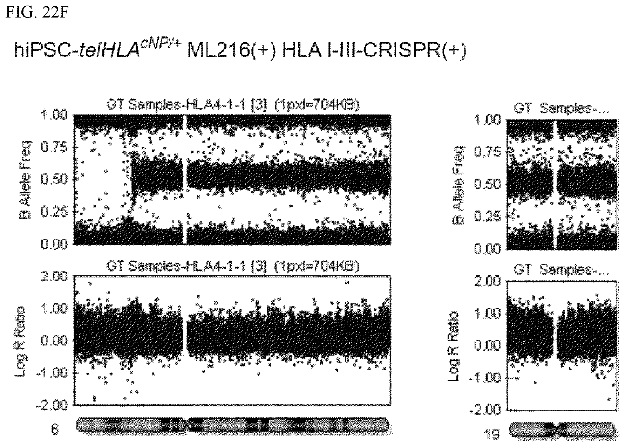

SNP array analysis results in chromosome 6 and chromosome 19 regarding parental fibroblast cells (parental fibroblast cells), hiPSCs, hiPSC-BLM^{tet/tet}AAVS1^{cNP/+}, hiPSC-BLM^{tet/tet}AAVS1^{cNP/+}Dox(+)9M-CRISPR(+), hiPSC-AAVS1^{cNP/+}ML216(+)9M-CRISPR(+), and hiPSC-telHLA^{cNP/+}ML216(+)HLA I-III-CRISPR(+)) are shown in Fig. 22A to Fig. 22F, respectively. As is apparent from Fig. 22A to Fig. 22E, when DSB is introduced into the position of 9 Mb in chromosome 19, a homozygous SNP pattern was observed for DSB on chromosome 19 in a telomere-like fashion from the results of hiPSC-BLM ^{tet/tet}AAVS1 ^{cNP/+}Dox(+)9M-CRISPR(+)(Fig. 22D) and hiPSC-AAVSl ^{cNP/+}ML216(+)9M-CRISPR(+)(Fig. 22E) in comparison with the parental fibroblast cells (parental fibroblast cells), hiPSCs, and hiPSC-BLM^{tet/tet}AAVS1^{cNP/+} (Fig. 22A to Fig. 22C). Also, as is apparent from Fig. 22A to Fig. 22C and Fig. 22F, when DSB is introduced at the position of HLA of chromosome 6, a homozygous SNP pattern was observed for DSB on chromosome 6 in a telomere-like fashion from the result of hiPSC-telHLA ^{cNP/+}ML216(+)HLA I-III-CRISPR(+)(Fig. 22F) in comparison with parental fibroblast cells (parental fibroblast cells), hiPSCs, and hiPSC-BLM^{tet/tet}AAVS1^{cNP/+} (Fig. 22A to Fig. 22C).

The aforementioned results of SNP array analysis revealed that chromosomal crossover occurred only at targeted positions. Although not depicted, it was confirmed that crossover did not occur in other chromosomes from the results of SNP array analysis for all the chromosomes other than chromosome 6 and chromosome 19. In particular, from the result of hiPSC-telHLA ^{cNP/+}ML216(+)HLA I-III-CRISPR(+)(Fig. 22F), it is expected that HLA homozygous hiPSC produced from parental fibroblast cells is useful for reducing the current drawback in hiPSC-based implantation caused by immunological difference between the dono and the host. That is, not only gene analysis of hiPSC is advanced, but also significant advance in regenerative medicine based on hiPSC can be expected.
SEQ ID NO: 1 is a gRNA targeting sequence for 34512783-34512803 of chromosome 19.
SEQ ID NO: 2 is a gRNA targeting sequence for 40498170-40498189 of chromosome 19.
SEQ ID NO: 3 is a gRNA targeting sequence for 44500571-44500590 of chromosome 19.
SEQ ID NO: 4 is a gRNA targeting sequence for 44536861-44536880 of chromosome 19.
SEQ ID NO: 5 is a gRNA targeting sequence for 2810497-2810516 of chromosome 6.

## Claims

1. A method for producing homozygous cells, comprising:
a step (A) of introducing DNA double strand break that is specific to one allele of homologous chromosomes into cells having a heterozygous mutation at a target site, and causing crossover in the presence of a Bloom's syndrome protein inhibitor to thereby obtain homozygous cells at the target site; and
a step (B) selecting the homozygous cells.

2. The method according to claim 1, wherein the Bloom's syndrome protein inhibitor is 1-(4-fluoro-3-(trifluoromethyl)phenyl)-3-(5-(pyridin-4-yl)1,3,4-thiadiazol-2-yl) urea and/or 1-(4-(1H-pyrazolyl)-3-cyanophenyl)-3-(5-(pyridin-4-yl)1,3,4-thiadiazol-2-yl) urea.

3. The method according to claim 1 or 2, wherein the Bloom's syndrome protein inhibitor is used in a concentration of 3.0 to 50.0 µM.

4. The method according to any one of claims 1 to 3, wherein the Bloom's syndrome protein inhibitor is made to coexist with the cells having a heterozygous mutation before the step (A).

5. The method according to any one of claims 1 to 4, wherein in the step (A), the DNA double strand break is conducted by a DNA double strand breaking method selected from a CRISPR/CRISPR-related nuclease method, a zinc finger nuclease (ZFN) method, or a transcription activator-like effector nuclease (TALEN) method.

6. The method according to any one of claims 1 to 5, wherein in the step (A), the introduction of the DNA double strand break that is specific to one allele of homologous chromosomes is conducted by introducing a vector designed by selecting a recognition sequence of which polymorphism exists in one allele from recognition sequences in the DNA double strand break, into the cells having a heterozygous mutation.

7. The method according to any one of claims 1 to 6, wherein
in the step (A), one or both of alleles of the homologous chromosomes is subjected to introduction of a selection cassette having such a sequence that a drug resistance gene to a first drug and a drug resistance gene to a second drug are arranged in directions opposite to each other, and inversion of the sequence, and
in the step (B), cells that are resistant to both of the first drug and the second drug are selected as homozygous cells at the target site.

8. The method according to claim 7, wherein
in the step (A), the selection cassette is introduced while being incorporated in a transposon vector, and
after the step (B), the selection cassette is removed by a re-excising transposase.

9. The method according to claim 7 or 8, wherein in the step (A), an insulator sequence is introduced on both sides or on either side of the selection cassette.

10. The method according to any one of claims 1 to 9, wherein the target site is a gene locus of a major histocompatibility antigen.

11. The method according to any one of claims 1 to 10, wherein the cells having a heterozygous mutation at the target site are diploid cells.

12. The method according to claim 11, wherein the diploid cells are human-derived cells.
